# EUROPEAN PATENT APPLICATION

(11) **EP 4 800 119 A1**
(43) Date of publication of application: **02.09.2026**
(21) Application number: 24897559.1
(22) Date of filing: 26.11.2024
(51) Int. Cl.: C12Q 1/68, C12N 5/10, C12N 15/09

(54) **SEARCH METHOD, CELL PRODUCTION METHOD, CELL SELECTION METHOD, CELL, AND METHOD FOR PRODUCING CELL PRODUCT**

(30) Priority: 29.11.2023 JP 2023202228
(71) Applicant: FUJIFILM Corporation, Tokyo 106-8620 (JP)
(72) Inventor: KOMATSU TANAKA, Yumiko, Ashigarakami-gun, Kanagawa 258-8577 (JP); SUGIYAMA, Satoshi, Ashigarakami-gun, Kanagawa 258-8577 (JP); HIKIDA, Yasushi, Ashigarakami-gun, Kanagawa 258-8577 (JP); NAKATANI, Keisuke, Ashigarakami-gun, Kanagawa 258-8577 (JP)
(74) Representative: Hoffmann Eitle
(86) International application number: PCT/JP2024/041880
(87) International publication number: WO 2025/115875

(57) **Abstract**

One aspect of a search method includes calculating a TAD score indicating a transcriptional activity for each TAD of a genome, and selecting a TAD based on the TAD score. Another aspect of the search method includes analyzing a genome acquired from a cell expressing an exogenous gene, and selecting a boundary of a region including the exogenous gene. A method for producing a cell includes inserting a target gene into a region found by the search method. A method for selecting a cell includes calculating a TAD score indicating a transcriptional activity for each TAD of a genome, selecting a TAD based on the TAD score, and selecting a cell in which a target gene is present in the selected TAD. The cell is a cell derived from a Chinese hamster, and is a cell in which a target gene is inserted into at least one predetermined region. A method for producing a cell product includes culturing a cell to express a target gene.

## Description

### BACKGROUND OF THE INVENTION

### 1. Field of the Invention

The present disclosure relates to a search method, a method for producing a cell, a method for selecting a cell, and a method for producing a cell and a cell product.

### 2. Description of the Related Art

EP2711428A discloses a site-specific integration host cell containing an endogenous Fer1L4 gene, in which an exogenous nucleotide sequence is integrated into the Fer1L4 gene.

WO2017/184831A discloses a cell comprising an exogenous nucleic acid integrated at a specific site in an expression-enhancing locus, in which the exogenous nucleic acid sequence encodes a bispecific antigen-binding protein.

WO2017/184832A discloses a cell comprising a first exogenous nucleic acid integrated into a first expression-enhancing locus and a second exogenous nucleic acid integrated into a second expression-enhancing locus, in which both the first and second exogenous nucleic acids encode an antigen-binding protein.

WO2020/072480A discloses a mammalian cell comprising a first recombinant target site (RTS) chromosomally integrated at a first high integration (HI) locus, in which the first HI locus is within about 30,000 base pairs of a genome compartment of active chromatin that is accessible and a TAD boundary, and the first HI locus overlaps with a region of a cell genome that interacts with at least one enhancer element.

### SUMMARY OF THE INVENTION

There is a technique for incorporating a target gene into a genome of a host cell for the purpose of creating a cell that stably produces a medical protein such as a humanized monoclonal antibody.

In a case where a region on a genome in which a gene is highly expressed or stably expressed is known in advance, a cell line in which the target gene is highly expressed or stably expressed can be produced with a high probability by inserting the target gene into the region.

The present disclosure has been made under the above circumstances.

An object of the present disclosure is to provide a search method for finding a target region on a genome, which is a target region for inserting a target gene and in which the target gene is highly expressed.

An object of the present disclosure is to provide a method for producing a cell that highly expresses a target gene.

Another object of the present disclosure is to provide a selection method for selecting a cell in which a target gene is highly expressed.

Another object of the present disclosure is to provide a cell that highly expresses a target gene.

An object of the present disclosure is to provide a method for producing a cell product having excellent productivity of a substance encoded by a target gene.

The specific means for achieving the objects includes the following aspects.
<1> A search method for finding a target region on a genome for inserting a target gene, the search method comprising:
   the following (1),
   in which the search method finds a TAD as the target region,
      (1) calculating, for each TAD included in the genome, a TAD score indicating transcriptional activity, and selecting a TAD based on the TAD score.
<2> The search method according to <1>, further comprising:
   the following (2);
   (2) for at least one known safe harbor in the genome, identifying a TAD score-SH that is the TAD score of a TAD to which the safe harbor belongs,
   determining a threshold value based on at least one TAD score-SH, and
   selecting a TAD based on the threshold value and the TAD score.
<3> A search method for finding a target region on a genome for inserting a target gene, the search method comprising:
   the following (a) to (d), in which the search method finds, as the target region, a region identified by a boundary pair P described below,
   (a) providing cells having a genome F in which an exogenous gene is integrated and expressing the exogenous gene,
   (b) obtaining the genome F from the cells, analyzing the genome F, and identifying a region F that is a region including the exogenous gene and a boundary F that is a boundary between the region F and the genome,
   (c) calculating, for each TAD included in the genome, a TAD score indicating transcriptional activity, identifying, for each boundary F, a TAD score-F that is the TAD score of a TAD to which the boundary F belongs, and selecting the boundary F based on the TAD score-F, and
   (d) finding, from among the selected boundaries F, a boundary pair P sandwiching the region F.
<4> The search method according to <3>, further comprising:
   the following (p), in which (c) is performed for the boundary F selected in the following (p),
   (p) measuring a methylation rate of the exogenous gene present in the region F and selecting a boundary F of the region F for which the methylation rate is low.
<5> The search method according to <3> or <4>, further comprising:
   the following (q), in which (d) is performed for the boundary F selected in the (c) and the following (q),
   (q) for at least one known safe harbor in the genome, identifying a TAD score-SH that is the TAD score of a TAD to which the safe harbor belongs,
   determining a threshold value based on at least one TAD score-SH, and
   selecting the boundary F based on the threshold value and the TAD score-F.
<6> The search method according to any one of <3> to <5>, further comprising:
   the following (r),
   (r) selecting, in the genome F, a boundary pair P in which the region F between the boundary pair P is not a region formed by chromosomal translocation.
<7> The search method according to any one of <1> to <6>, in which the TAD score is a value obtained by multiplying a density of genes present in the TAD by an average expression level.
<8> The search method according to any one of <1> to <7>, in which the genome is a genome of a mammalian cell.
<9> The search method according to any one of <1> to <7>, in which the genome is a genome of a CHO cell.
<10> A method for producing a cell, the method comprising:
   finding the TAD by the search method according to <1> or <2>; and
   inserting a target gene into an inside of the TAD in a genome having the TAD.
<11> A method for producing a cell, the method comprising:
   finding a region identified by the boundary pair P by the search method according to any one of <3> to <9>; and
   inserting a gene of interest into region within ±10 kbp before and after the region in a genome having the region.
<12> The method for producing a cell according to <10> or <11>, in which the target gene is a gene encoding at least one selected from the group consisting of an enzyme, an antibody, an interleukin, a cytokine, a chemokine, a hormone, a growth factor, a transcription factor, a receptor, a transcription-regulatory nucleic acid, a non-coding RNA, a viral preparation, a vaccine, a therapeutic protein, subunits thereof, and fragments thereof.
<13> A method for selecting a cell, which is a method for selecting a cell expressing a gene of interest, the method comprising:
   the following (11)
   (11) calculating, for each TAD included in a genome of a cell, a TAD score indicating transcriptional activity, selecting a TAD based on the TAD score, and selecting a cell in which the target gene is present in the selected TAD.
<14> The method for selecting a cell according to <13>, the method further comprising:
   the following (12),
   (12) for at least one known safe harbor in the genome, identifying a TAD score-SH that is the TAD score of a TAD to which the safe harbor belongs,
   determining a threshold value based on at least one TAD score-SH, and
   selecting a TAD based on the threshold value and the TAD score and selecting a cell in which the target gene is present in the selected TAD.
<15> The method for selecting a cell according to <13> or <14>, the method further comprising:
   the following (13),
   (13) measuring a methylation rate of the target gene present in the selected TAD, and selecting a cell having a low methylation rate.
<16> The method for selecting a cell according to any one of <13> to <15>, in which the TAD score is a value obtained by multiplying a density of genes present in the TAD by an average expression level.
<17> The method for selecting a cell according to any one of <13> to <16>, in which the cell is a mammalian cell.
<18> The method for selecting a cell according to any one of <13> to <16>, in which the cell is a CHO cell.
<19> The method for selecting a cell according to any one of <13> to <18>, in which the target gene is a gene encoding at least one selected from the group consisting of an enzyme, an antibody, an interleukin, a cytokine, a chemokine, a hormone, a growth factor, a transcription factor, a receptor, a transcription-regulatory nucleic acid, a non-coding RNA, a viral preparation, a vaccine, a therapeutic protein, subunits thereof, and fragments thereof.
<20> A cell derived from a Chinese hamster, in which a target gene is inserted into at least one region selected from 40 regions shown in Table 1 below.
<21> A cell derived from a Chinese hamster, in which a target gene is inserted into at least one region selected from 40 regions shown in Table 2 below.
<22> A cell derived from a Chinese hamster, in which a target gene is inserted into at least one region selected from 40 regions shown in Table 3 below.
<23> The cell according to any one of <20> to <22>, in which cell derived from a Chinese hamster is a CHO cell.
<24> The cell according to any one of <20> to <23>, in which the target gene is a gene encoding at least one selected from the group consisting of an enzyme, an antibody, an interleukin, a cytokine, a chemokine, a hormone, a growth factor, a transcription factor, a receptor, a transcription-regulatory nucleic acid, a non-coding RNA, a viral preparation, a vaccine, a therapeutic protein, subunits thereof, and fragments thereof.
<25> A method for producing a cell, the method comprising:
   inserting a target gene into at least one region selected from 40 regions shown in Table 1 of a genome of a cell derived from a Chinese hamster.
<26> A method for producing a cell, the method comprising:
   inserting a target gene into at least one region selected from 40 regions shown in Table 2 of a genome of a cell derived from a Chinese hamster.
<27> A method for producing a cell, the method comprising:
   inserting a target gene into at least one region selected from 40 regions shown in Table 3 of a genome of a cell derived from a Chinese hamster.
<28> The method for producing a cell according to any one of <25> to <27>, in which the cell derived from a Chinese hamster is a CHO cell.
<29> The method for producing a cell according to any one of <25> to <28>, in which the target gene is a gene encoding at least one selected from the group consisting of an enzyme, an antibody, an interleukin, a cytokine, a chemokine, a hormone, a growth factor, a transcription factor, a receptor, a transcription-regulatory nucleic acid, a non-coding RNA, a viral preparation, a vaccine, a therapeutic protein, subunits thereof, and fragments thereof.
<30> A method for producing a cell product, the method comprising:
   culturing a cell produced by the method for producing a cell according to any one of <10> to <12> and <25> to <29> to express the target gene.
<31> A method for producing a cell product, the method comprising:
   culturing a cell produced by the method for producing a cell according to any one of <13> to <19> to express the target gene.
<32> A method for producing a cell product, the method comprising: culturing a cell produced by the method for producing a cell according to any one of <20> to <24> to express the target gene.
<33> The manufacturing method for a cell product according to any one of <30> to <32>, in which the target gene is a gene encoding at least one selected from the group consisting of an enzyme, an antibody, an interleukin, a cytokine, a chemokine, a hormone, a growth factor, a transcription factor, a receptor, a transcription-controlling nucleic acid, a non-coding RNA, a viral preparation, a vaccine, a therapeutic protein, a subunit thereof, and a fragment thereof.

According to the present disclosure, there is provided a search method for finding a target region on a genome, into which a target gene is inserted, in which the target gene is highly expressed.

According to the present disclosure, there is provided a method for producing a cell in which a target gene is highly expressed.

According to the present disclosure, there is provided a selection method for selecting a cell in which a target gene is highly expressed.

According to the present disclosure, there is provided a cell in which a target gene is highly expressed.

According to the present disclosure, there is provided a manufacturing method for a cell product having excellent productivity of a substance encoded by a target gene.

### BRIEF DESCRIPTION OF THE DRAWINGS

FIG. 1 is a conceptual diagram of a second search method.
FIG. 2 is a scatter plot showing antibody production performance of 60 antibody-producing cell lines produced in Examples.
FIG. 3 is a histogram of copy numbers of exogenous genes present inside a pair of boundaries, which is obtained from genome analysis of 31 antibody-producing cell lines.
FIG. 4 is a histogram of average methylation rates of a promoter region and an antibody subunit region, which is obtained from genome analysis of 31 antibody-producing cell lines.
FIG. 5 is an example of a contact map of a CHO cell.
FIG. 6 is a distribution diagram of TAD scores related to 2,502 TADs detected in a genome of a CHO cell.
FIG. 7 is a scatter plot showing performance of a high-performance region found by the present embodiment.
FIG. 8 is a graph showing performance of the high-performance region found by the present embodiment.
FIG. 9 is a graph showing that a multi-copy insertion of a coding sequence is possible in the high-performance region found by the present embodiment.
FIG. 10 is a graph showing that the high-performance region found by the present embodiment can stably express a multi-copy coding sequence.

### DESCRIPTION OF THE PREFERRED EMBODIMENTS

Hereinafter, embodiments according to the present disclosure will be described. These descriptions and Examples are only illustrative of the embodiments and do not limit the scope of the embodiments. The action mechanism mentioned in the present disclosure includes estimation, and the accuracy thereof does not limit the scope of the embodiments.

In a case where the embodiments of the present disclosure are described with reference to the drawings, the configurations of the embodiments of the present disclosure are not limited to the configurations shown in the drawings. The sizes of the elements in the drawings are conceptual, and the relative relationship between the sizes of the elements is not limited thereto.

In the present disclosure, the term "step" includes not only an independent step, but also a step that may not be clearly distinguished from other steps but still achieves a desired effect of the step.

In the present disclosure, a numerical range expressed using "to" indicates a range including numerical values before and after "to" as a minimum value and a maximum value.

In a numerical range described in a stepwise manner in the present disclosure, an upper limit or a lower limit described in one numerical range may be replaced with an upper limit or a lower limit in another numerical range described in a stepwise manner. In addition, in the numerical ranges described in the present disclosure, the upper limit value or the lower limit value of the numerical range may be replaced with the value shown in Examples.

In the present disclosure, each component may contain a plurality of kinds of substances corresponding thereto. In the present disclosure, upon referring to an amount of each component in a composition, the amount means a total amount of a plurality of types of substances present in the composition unless otherwise specified, in a case where a plurality of types of substances corresponding to each component are present in the composition.

In the present disclosure, the term "nucleic acid" includes all nucleic acids (for example, deoxyribonucleic acid (DNA), ribonucleic acid (RNA), analogs thereof, natural products, and artificial products), as well as all nucleic acids to which low-molecular-weight compounds, groups (for example, methyl groups), non-nucleic acid molecules, structures, etc. are linked. The nucleic acid may be single-stranded or double-stranded.

In the present disclosure, the donor vector is a substance having an action of introducing an exogenous nucleic acid into a cell and a genome of the cell, and is a nucleic acid itself. The origin, the form, and the base sequence of the donor vector are not limited. The donor vector may be a cyclic nucleic acid or a linear nucleic acid. The donor vector may be a single-stranded nucleic acid or a double-stranded nucleic acid. The donor vector is preferably a double-stranded DNA.

In the present disclosure, the number of amino acid residues of the protein is not limited. The protein includes a protein in which an amino acid is post-translationally modified. Examples of the post-translational modification of an amino acid include phosphorylation, methylation, acetylation, glycosylation, lipidation, and the like.

In the present disclosure, in the notation of an amino acid, the three-letter notation and the one-letter notation established by IUPAC-IUBMB joint commission on Biochemical Nomenclature (IUPAC-IUBMB JCBN) are used. Unless otherwise specified, the amino acid referred to in the present disclosure is an L-amino acid.

In the present disclosure, the identity of the base sequence and the identity of the amino acid sequence are calculated using a basic local alignment search tool (BLAST) (https://blast.ncbi.nlm.nih.gov/Blast.cgi).

A topologically associating domain (TAD) is a structural unit in a genome detected by three-dimensional genome structure analysis, and is a region having a relatively high spatial contact probability. The size of the TAD is generally several hundred kbp to several Mbp.

The algorithm for the three-dimensional genome structure analysis may be an algorithm that has been published, an algorithm that has been improved from the published algorithm, or a newly developed algorithm.

The genome structure data for the three-dimensional genome structure analysis may be data acquired from a published database, an academic paper, a technical literature, or the like, or may be data obtained by actually analyzing an intracellular genome.

Examples of the analysis method for the three-dimensional genome structure analysis include a Hi-C method; an in situ Hi-C method, a low Hi-C method, a SAFE Hi-C method, and a Hi-CO method, which are based on the Hi-C method as a basic principle; and a Micro-C method.

The Hi-C method is one of the analysis methods for the three-dimensional genome structure analysis, and is an analysis method for comprehensively detecting regions spatially close to each other in the genome over the entire genome. Hi-C is an abbreviation for high-throughput chromosome conformation capture.

From the calculation results of the Hi-C analysis, a region having a relatively high spatial contact probability, that is, a TAD is detected, and the genome is compartmentalized into a plurality of TADs. The size of the TAD detected by the Hi-C analysis is generally several hundred kbp to several Mbp. By the Hi-C analysis, a mammalian genome is compartmentalized into several thousand TADs.

The algorithm for the Hi-C analysis used for the detection of the TAD may be an algorithm that has been published, an algorithm that has been improved from the published algorithm, or a newly developed algorithm.

The genome structure data for the Hi-C analysis may be data acquired from a published database, an academic paper, a technical literature, or the like, or may be data obtained by actually analyzing an intracellular genome.

The safe harbor in the genome is a region in which a host cell survives even in a case where a gene is inserted, and is a region in which the inserted gene is expressed. The safe harbor in the genome is specified by a chromosome number or an accession number and a base number of a public base sequence database. Examples of the public base sequence database include the International Nucleotide Sequence Databases (INSD), RefSeq (NCBI Reference Sequence Database), and the like.

The safe harbor in the genome may be referred to by the name of a known gene present in the region or in the vicinity of the region.

### <Search method>

The present disclosure provides a search method for finding a target region on a genome into which a target gene is to be inserted.

The present disclosure provides a first search method and a second search method.

### <First search method>

The target region found by the first search method is a TAD which is a structural unit in the genome and is a TAD having a relatively high transcriptional activity.

The TAD includes a TAD having a relatively high transcriptional activity and a TAD having a relatively low transcriptional activity. The first search method includes the following (1) for the purpose of finding a TAD having a relatively high transcriptional activity.

(1) A TAD score indicating a transcriptional activity is calculated for each TAD in the genome, and a TAD is selected based on the TAD score.

In the present disclosure, the TAD score is an evaluation standard indicating a high or low level of transcriptional activity. The TAD score is usually a preferable evaluation standard as the higher the TAD score is.

The selection of the TAD based on the TAD score is, for example, the selection of a TAD having a relatively high TAD score; or the selection of a TAD having a TAD score exceeding a predetermined criterion.

The TAD score may be an indicator for finding a TAD having a relatively high transcriptional activity from among a large number of TADs found on the genome (for example, a mammalian genome is partitioned into thousands of TADs).

The TAD score is, for example, a total amount, an average value, or a median value of expression levels of genes present in the TAD, a density of genes present in the TAD, a reciprocal of a methylation rate or an unmethylation rate of CpG sites in the TAD, a value obtained by multiplying two or more of these values, or an indicator based on two or more of these values.

The genes present in the TAD can be known from publicly available databases, academic papers, technical documents, and the like. The genes present in the TAD and involved in the calculation of the TAD score are at least one gene, preferably two or more genes, and the larger the number of genes is, the more preferable it is, and it is preferable to cover as many genes present in the TAD as possible.

The expression level of the gene may be data acquired from publicly available databases, academic papers, technical documents, and the like, or may be data obtained by actually quantifying the expression level of the gene. The quantification of the expression level of the gene can be performed by a known mRNA quantification method.

An example of the embodiment of the TAD score is a value obtained by multiplying the density of genes present in the TAD by the average expression level. The higher the value is, the more preferable the evaluation standard. The density of genes present in the TAD is a value (genes/Mb) obtained by dividing the number of endogenous genes present in the TAD by the number of base pairs of the TAD.

An example of the embodiment of the first search method further includes the following (2).

(2) specifying a TAD score-SH, which is a TAD score of a TAD to which at least one known safe harbor in a genome belongs,
determining a threshold value based on at least one TAD score-SH, and
selecting TAD based on the threshold value and the TAD score-SH.

The known safe harbor in the genome can be known from publicly available databases, academic papers, technical documents, and the like. The number of safe harbors for specifying the TAD score-SH is at least one, preferably two or more, and for example, eight or less.

In a case where there are a large number of known safe harbors, at least one may be selected. Examples of a method of selecting the safe harbor include selecting a safe harbor in which the expression level (pg/cell/copy) of a protein encoded by the inserted gene is relatively high; and selecting a safe harbor in which the expression level (pg/cell/copy) of a protein encoded by the inserted gene exceeds a predetermined criterion. The protein expression level of the safe harbor may be data acquired from a publicly available database, an academic paper, a technical literature, and the like, or may be data obtained by actually inserting a gene into the safe harbor and measuring the protein expression level.

The coordinates of the safe harbor (that is, the chromosome number or the accession number and the base number of the public base sequence database) are applied to the compartment of the TAD (which is the output of the three-dimensional genome structure analysis (for example, Hi-C analysis)), the TAD to which the safe harbor belongs is specified, and then the TAD score of the TAD is specified. Here, the TAD score is the TAD score calculated for each TAD in (1).

In the present disclosure, the TAD score of the TAD to which the safe harbor belongs is referred to as a "TAD score-SH". At least one TAD score-SH is specified.

The threshold value based on the TAD score-SH is, for example, the minimum value, the maximum value, the average value, or the median value of at least one TAD score-SH.

The TAD score is usually a preferable evaluation standard as the higher the TAD score is. The selection of the TAD based on the threshold value and the TAD score is the selection of the TAD having a TAD score exceeding the threshold value.

The TAD selected based on the threshold value based on the TAD score-SH is expected to be equivalent to the known safe harbor or to exhibit a gene expression level that exceeds the known safe harbor.

Even in a case where (1) and (2) cannot be clearly distinguished from each other, the first search method includes (1) and (2) as long as the purposes of (1) and (2) are achieved.

### <Second search method>

The target region found by the second search method is a safe harbor in the genome and is a region having a relatively high transcriptional activity.

The second search method includes the following (a) to (d) for the purpose of finding a safe harbor in the genome and a region having a relatively high transcriptional activity, and finds a region specified by a boundary pair P. The region specified by the boundary pair P is a region having the boundary pair P as a start point and an end point.
(a) Preparing a cell having a genome F in which an exogenous gene is incorporated into a genome and expressing the exogenous gene.
(b) Acquiring the genome F from the cell, analyzing the genome F, and specifying a region F which is a region including the exogenous gene and a boundary F which is a boundary between the region F and the genome.
(c) Calculating a TAD score indicating a transcriptional activity for each TAD of the genome, specifying a TAD score-F which is a TAD score of a TAD to which the boundary F belongs, for each boundary F, and selecting the boundary F based on the TAD score-F.
(d) Finding a boundary pair P that sandwiches the region F from the selected boundaries F.

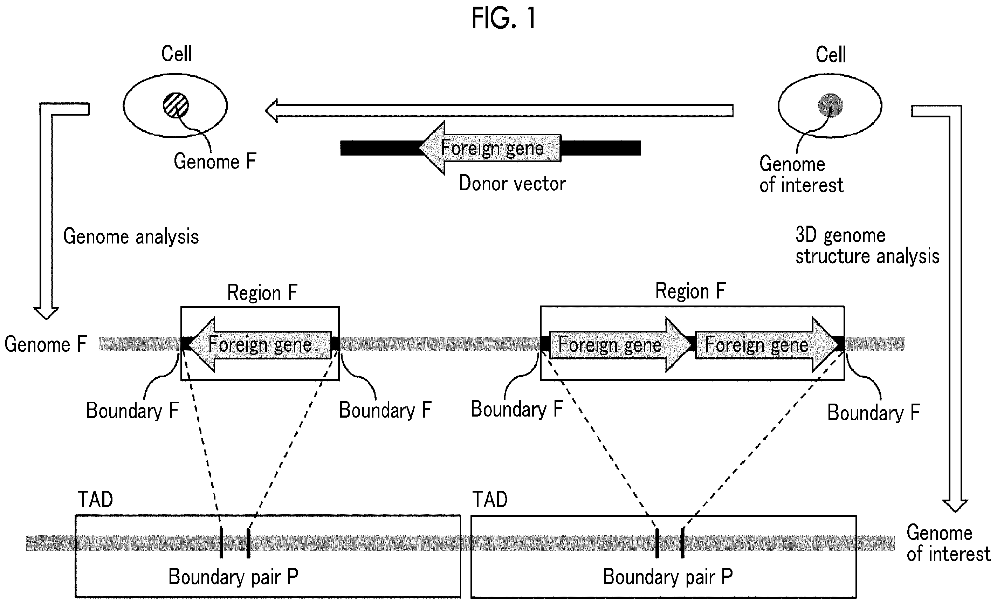

FIG. 1 is a conceptual diagram of the second search method. FIG. 1 shows a relationship between a genome, an exogenous gene, a cell, a genome F, a region F, a boundary F, a TAD, and a boundary pair P, which are targets of the second search method.

The exogenous gene in (a) and (b) is a gene that is not originally included in the genome which is a target of the second search method, and is a gene that is focused on in a case of genome analysis for the purpose of finding a safe harbor. The origin, the type, the size, and the base sequence of the exogenous gene are not limited. An example of an embodiment of the exogenous gene is a structural gene (that is, a gene encoding a protein).

In the present disclosure, the exogenous gene means an exogenous gene that can be expressed in a cell. The exogenous gene has all sequences necessary for the expression of the exogenous gene. In a case where the exogenous gene is a structural gene, the exogenous gene has all sequences necessary for the expression of a protein encoded by the exogenous gene, and includes a coding sequence of the protein and all nucleic acids (for example, a promoter, a transcription terminator, and a polyadenylation sequence) necessary for transcription and translation of the coding sequence in a cell. The exogenous gene may include one copy of the coding sequence of the protein, or may include two or more copies thereof. For example, the exogenous gene may include at least one copy of a coding sequence of each subunit in order to express all subunits of a heteromultimeric protein. For example, the exogenous gene may include at least one copy of a sequence encoding a heavy chain of an antibody and a sequence encoding a light chain of the antibody.

The cell in (a) may be an already established established cell line or a newly created cell. The cell may be a polyclonal cell population or a monoclonal cell.

Since the cell in (a) is a cell in which the exogenous gene is incorporated into the genome without being killed and the exogenous gene is expressed, the safe harbor can be found by analyzing the genome F acquired from the cell.

A preferred example of the cell in (a) is a monoclonal cell in which the exogenous gene is stably highly expressed. The region of the genome F of the cell, in which the exogenous gene is inserted, is presumed to be a high-performance safe harbor (that is, a region in which the host cell survives even after the gene is inserted and the inserted gene is stably highly expressed). By analyzing the genome F acquired from the cell, a high-performance safe harbor can be efficiently found.

Specifically, (b) includes, for example, genome extraction from a cell, preparation of a sequencing library, library sequencing, mapping of reads onto a genome, mapping of an exogenous gene onto a read, specification of a boundary between the genome and the exogenous gene, extraction of a read including the exogenous gene, measurement of the copy number of the exogenous gene included in the read, and specification of a boundary pair sandwiching the exogenous gene.

From the viewpoint of acquiring a long read and the viewpoint of obtaining DNA methylation data, the library sequencing is preferably single-molecule real-time sequencing or nanopore sequencing.

The region F is a region in the genome F, is a region distinguished from the genome, and is a region including at least one copy of the exogenous gene.

The boundary F is a boundary between the region F in the genome F and the genome. The coordinates of the boundary F are specified by a chromosome number of the genome, which is a search target, or an accession number and a base number of a public base sequence database. Examples of the public base sequence database include the International Nucleotide Sequence Databases (INSD), RefSeq (NCBI Reference Sequence Database), and the like.

An example of the embodiment of (b) includes narrowing down the region F to a region including a multi-copy (that is, two or more copies) of the exogenous gene. In this case, the boundary F of the region F including the multi-copy of the exogenous gene is a target of (c). The copy number of the exogenous gene included in the region F may be, for example, 2 copies to 6 copies, 2 copies to 5 copies, or 2 copies to 4 copies.

Since the region F including the multi-copy of the exogenous gene has multi-copy resistance, a target substance can be stably produced even in a case where a target gene of a relatively long base length is inserted in a multi-copy manner. That is, a high production amount of the target substance is maintained even after long-term culture. In the related art, in a case where a multi-copy of an exogenous gene is inserted at one site, a production amount of a substance encoded by the exogenous gene tends to be unstable, but this problem is improved according to the embodiment of the present disclosure.

Examples of the target gene of a relatively long base length in a multi-copy manner include a nucleic acid in which coding sequences are linked in two or more copies, and a nucleic acid in which coding sequences of all subunits of a heteromultimeric protein are linked in at least one copy. A region in which the nucleic acid in which the coding sequences are linked in two or more copies can be inserted is a region in which a substance encoded by the target gene can be highly produced. A region in which the nucleic acid in which the coding sequences of all subunits of the heteromultimeric protein are linked in at least one copy can be inserted is a region in which the heteromultimeric protein can be stably produced.

The TAD and the TAD score in (c) have the same meaning as the TAD and the TAD score in (1) of the first search method, and the specific form and the calculation method are also the same.

An example of the embodiment of the TAD score is a value obtained by multiplying the density of the gene present in the TAD by the average expression level. The higher the value is, the more preferable the evaluation standard.

The coordinates of the boundary F (that is, the chromosome number or the accession number and the base number of the public base sequence database) are applied to the compartment of the TAD (output of the three-dimensional genome structure analysis (for example, Hi-C analysis)), the TAD to which the boundary F belongs is specified, and then the TAD score of the TAD is specified. Here, the TAD score is a TAD score calculated for each TAD.

In the present disclosure, the TAD score of the TAD to which the boundary F belongs is referred to as a "TAD score-F".

The TAD score is usually a preferable evaluation standard as the higher the TAD score is. The selection of the boundary F based on the TAD score-F is, for example, the selection of the boundary F having a relatively high TAD score-F; or the selection of the boundary F having a TAD score-F exceeding a predetermined criterion.

(d) is to find a boundary pair P that sandwiches the region F from among the boundaries F selected in (c). The boundary pair P is one end and the other end of the same region F in the genome F.

Since the boundary pair P is a pair of the boundaries F selected in (c), the boundary pair P is a coordinate pair present in the TAD having a relatively high transcriptional activity. Therefore, it is expected that the region specified by the boundary pair P (that is, the region having the boundary pair P as a start point and an end point) is a region having a relatively high transcriptional activity.

The coordinate pair of the boundary pair P is specified by a chromosome number of the genome, which is a search target, or an accession number and a base number of a public base sequence database. Examples of the public base sequence database include the International Nucleotide Sequence Databases (INSD), RefSeq (NCBI Reference Sequence Database), and the like.

In (b), in a case where the region F is narrowed down to a region including multiple copies of an exogenous gene, the region specified by the boundary pair P can stably produce a target substance even in a case where a target gene having a relatively long base length (for example, a nucleic acid in which coding sequences of two or more copies are linked, or a nucleic acid in which coding sequences of all subunits of a heteromultimeric protein are linked by at least one copy) is inserted. That is, a high production amount of the target substance is maintained even after long-term culture.

An example of an embodiment of the second search method further includes the following (p), and (c) is performed on the boundary F selected by (p).

(p) Measuring a methylation rate of an exogenous gene present in the region F and selecting the boundary F of the region F having a low methylation rate.

DNA methylation generally suppresses gene expression. Therefore, (p) is to select the region F in which gene expression is not suppressed and the boundary F thereof. By performing (c) on the boundary F selected by (p) and then performing (d), the boundary pair P related to the region in which high expression of the gene can be expected is found.

The methylation rate of the exogenous gene can be acquired from data obtained by performing single-molecule real-time sequencing or nanopore sequencing as the library sequencing in (b).

The value of the methylation rate is, for example, methylated cytosine at the CpG site/total cytosine at the CpG site × 100.

The value of the methylation rate may be a value of the entire exogenous gene, a value of a part of the exogenous gene (for example, a value of a sequence encoding a protein), or a value for each range in which the exogenous gene is divided according to the function (for example, a value of each of a promoter region and a sequence encoding a protein). The minimum value, the maximum value, the average value, or the median value of at least one methylation rate obtained from the target range is used as a representative value for selecting the region F and the boundary F thereof.

The selection of the region F in which the methylation rate is a low value is, for example, the selection of the region F in which the methylation rate is relatively low; or the selection of the region F in which the methylation rate is lower than a predetermined criterion (for example, 30%, 20%, or 10%).

An example of the second search method further includes the following (q), and (d) is performed on the boundary F selected by (c) and (q).

(q) specifying a TAD score-SH, which is a TAD score of a TAD to which at least one known safe harbor in the genome belongs,
determining a threshold value based on at least one TAD score-SH,
and selecting the boundary F based on the threshold value and the TAD score-F.

The known safe harbor in the genome, the TAD score-SH, and the threshold value in (q) have the same meanings as the known safe harbor in the genome, the TAD score-SH, and the threshold value in (2) of the first search method, and specific forms and specific methods thereof are also the same.

The TAD score is usually a preferable evaluation standard as the higher the TAD score is. The selection of the boundary F based on the threshold value and the TAD score-F is the selection of the boundary F having the TAD score-F exceeding the threshold value.

The boundary F is selected by performing (c) and (q), and then the boundary pair P related to the region expected to exhibit the gene expression level equal to or higher than the gene expression level of the known safe harbor is found by performing (d).

Even in a case where (c) and (q) cannot be clearly distinguished from each other, the second search method includes (c) and (q) as long as the purpose of (c) and (q) is achieved.

An example of the embodiment of the second search method further includes the following (r).

(r) selecting the boundary pair P in which the region F between the boundary pair P in the genome F is not a region formed by a chromosomal translocation.

The confirmation that the region F is not a region formed by a chromosomal translocation is performed, for example, by confirming that one coordinate and the other coordinate of the boundary pair P sandwiching the region F belong to the same chromosome and that the base length of the region F (in other words, the distance between the boundary pair P on the genome F) is not too long. For example, in a case where the base length of the region F is 100 kbp or less, it is determined that the region F is not a region formed by a chromosomal translocation, and the boundary pair P sandwiching the region F is selected.

Even in a case where (d) and (r) cannot be clearly distinguished from each other, the second search method includes (d) and (r) as long as the purpose of (d) and (r) is achieved.

The genome that is a target of the first search method is a genome of any cell as long as the genome has a TAD, which is a structural unit in the genome. Examples of the cell include fungi, yeast, insect cells, mammalian cells, and plant cells.

The genome that is a target of the second search method is a genome of any cell as long as the genome satisfies the condition of having a TAD, which is a structural unit in the genome, and the condition of being able to produce a cell in which a gene is incorporated into the genome and the gene is expressed. Examples of the cell include fungi, yeast, insect cells, mammalian cells, and plant cells.

Examples of the fungi include Aspergillus oryzae.

Examples of the yeast include budding yeast (Saccharomyces cerevisiae), Pichia pastoris, and Hansenula polymorpha.

Examples of the insect cell include a BmN cell derived from silkworm (Bombyx mori), an Sf9 cell and an Sf21 cell derived from cabbage armyworm (Spodoptera frugiperda), an S2 cell derived from fruit fly (Drosophila melanogaster), and Pv11 cells derived from sleeping chironomid (Polypedilum vanderplanki).

Examples of the mammalian cell include Chinese hamster ovary cells (CHO cells), baby hamster kidney cells (BHK cells), a human embryonic kidney cell line (for example, HEK293 cells), a human retinoblast-derived cell line (for example, PER.C6 cells), a mouse myeloma cell line (for example, NS0 cells and SP2/0 cells), and established cell lines derived from these cells.

Examples of the CHO cell include a CHO-DG44 cell, a CHO-K1 cell, a CHO-DXB11 cell, a CHOpro3⁻ cell, and an established cell derived from these cells.

Examples of the mammalian cell include cells having a differentiation ability into other cells. Examples thereof include pluripotent stem cells such as embryonic stem cells (ES cells) and induced pluripotent stem cells (iPS cells); multipotent stem cells such as mesenchymal stem cells, tissue stem cells, and somatic stem cells; and the like.

### <Method for Producing a Cell>

The present disclosure provides a method for producing a cell in which a target gene is highly expressed.

The present disclosure provides a cell production method including a first search method and a cell production method including a second search method.

The cell production method including the first search method includes finding a TAD by the first search method and inserting a target gene into the inside of the TAD of a genome having the TAD.

In the cell production method including the first search method, the insertion region of the target gene is the inside of the TAD selected based on the TAD score indicating the transcriptional activity, that is, the inside of the TAD having a relatively high transcriptional activity. Therefore, the produced cell has a high possibility of highly expressing the target gene.

The cell production method including the second search method includes finding a region specified by a boundary pair P by the second search method and inserting a target gene into the inside of the region of ±10 kbp before and after the region of a genome having the region.

In the cell production method including the second search method, the insertion region of the target gene may be the inside of the region specified by the boundary pair P (that is, the region having the boundary pair P as a start point and an end point), may be the inside of the region having the boundary pair P as a start point and an end point, may be the outside of the region having the boundary pair P as a start point and an end point, or may be across the inside and the outside of the region having the boundary pair P as a start point and an end point, as long as the insertion region is the inside of ±10 kbp before and after the region specified by the boundary pair P. Since the insertion region of the target gene is the inside of the safe harbor found by the second search method or the vicinity thereof, the produced cell has a high possibility of highly expressing the target gene without being killed.

In the cell production method including the second search method, the insertion region of the target gene may be limited to a narrower range. Examples of the insertion region of the target gene include the inside of ±8 kbp before and after the region specified by the boundary pair P; the inside of ±5 kbp before and after the region specified by the boundary pair P; the inside of ±3 kbp before and after the region specified by the boundary pair P; the inside of ±1 kbp before and after the region specified by the boundary pair P; and the inside of the region specified by the boundary pair P.

The genome into which the target gene is to be inserted is any genome of a cell as long as the genome has a TAD, which is a structural unit in the genome. Examples of the cell include fungi, yeast, insect cells, mammalian cells, and plant cells. Specific examples of the cell are the same as the specific examples described in the description of the search method of the present disclosure.

An example of the embodiment of the cell production method of the present disclosure is to insert a target gene into a genome of a mammalian cell. Examples of the mammalian cell include Chinese hamster ovary cells (CHO cells), baby hamster kidney cells (BHK cells), a human embryonic kidney cell line (for example, HEK293 cells), a human retinoblast-derived cell line (for example, PER.C6 cells), a mouse myeloma cell line (for example, NS0 cells and SP2/0 cells), and established cell lines derived from these cells.

An example of the embodiment of the cell production method of the present disclosure is to insert a target gene into a genome of a CHO cell. Examples of the CHO cell include a CHO-DG44 cell, a CHO-K1 cell, a CHO-DXB11 cell, a CHOpro3⁻ cell, and an established cell derived from these cells.

An example of the embodiment of the cell production method of the present disclosure is to insert a target gene into a genome of a cell having a differentiation ability into other cells. Examples of the cell include pluripotent stem cells such as ES cells and iPS cells; and multipotent stem cells such as mesenchymal stem cells, tissue stem cells, and somatic stem cells.

The insertion of the target gene into the target region of the genome is possible by a known genome editing technology.

The origin, the size, and the base sequence of the target gene are not limited. The target gene includes a nucleic acid encoding a protein and a nucleic acid not encoding a protein.

Examples of the target gene include a gene encoding at least one selected from the group consisting of an enzyme, an antibody, an interleukin, a cytokine, a chemokine, a hormone, a growth factor, a transcription factor, a receptor, a transcription-controlling nucleic acid, a non-coding RNA, a viral preparation, a vaccine, a therapeutic protein, a subunit thereof, and a fragment thereof.

In a case where the target gene is a nucleic acid encoding a protein, examples of the protein encoded by the target gene (referred to as a "target protein" in the present disclosure) include at least one selected from the group consisting of an enzyme, an antibody, an interleukin, a cytokine, a chemokine, a hormone, a growth factor, a transcription factor, a receptor, a protein constituting a viral preparation, a vaccine, a therapeutic protein, a subunit thereof, and a fragment thereof.

In the present disclosure, the antibody is not limited to an immunoglobulin and may be any molecule that binds to an antigen. In the present disclosure, the antibody is a term includes an antibody fragment and an antigen-binding molecule. In the present disclosure, the heavy chain of the antibody is also referred to as an H chain, and the light chain of the antibody is also referred to as an L chain.

Examples of the nucleic acid not encoding a protein include a transcription-controlling nucleic acid, a non-coding RNA, and a nucleic acid constituting a viral preparation.

Examples of the non-coding RNA (ncRNA) include microRNA (miRNA), short hairpin RNA (shRNA), small interfering RNA (siRNA), small nuclear RNA (snRNA), ribosomal RNA (rRNA), and transfer RNA (tRNA).

In the present disclosure, the target gene means a target gene that can be expressed in a cell. The target gene has all sequences necessary for the expression of the target gene. In a case where the target gene is a structural gene, the target gene has all sequences necessary for the expression of a protein encoded by the target gene, and includes a coding sequence of the protein and all nucleic acids (for example, a promoter, a transcription terminator, and a polyadenylation sequence) necessary for transcription and translation of the coding sequence in a cell. The target gene may include one copy of a coding sequence of a protein, or may include two or more copies thereof. For example, the target gene may include at least one copy of a coding sequence of each subunit in order to express all subunits of a heteromultimeric protein. For example, the target gene may have at least one copy of a sequence encoding an H chain of an antibody and at least one copy of a sequence encoding an L chain of the antibody.

An example of an embodiment of the cell production method according to the present disclosure is to insert a nucleic acid in which two or more copies of a coding sequence are linked, into a target region of a genome as a target gene. According to the present embodiment, a cell capable of highly producing a substance encoded by a target gene is produced.

From the viewpoint of long-term subculture stability of the cell, the number of copies of the coding sequence included in the target gene is preferably not too large, and is preferably 2 to 6, more preferably 2 to 5, and still more preferably 2 to 4.

An example of an embodiment of the cell production method according to the present disclosure is to insert a nucleic acid in which at least one copy of a coding sequence of each subunit of a heteromultimeric protein is linked, into a target region of a genome as a target gene. According to the present embodiment, a cell capable of stably producing a heteromultimeric protein is produced.

From the viewpoint of increasing the expression level of the heteromultimeric protein, the target gene in the present embodiment is preferably a nucleic acid in which two or more copies of a set of coding sequences of all subunits are linked. From the viewpoint of long-term subculture stability of the cell, the number of copies of the set included in the target gene is preferably not too large, and is preferably 2 to 6, more preferably 2 to 5, and still more preferably 2 to 4.

### <Method for selecting cell>

The present disclosure provides a method for selecting a cell that highly expresses a target gene.

The origin, the size, and the base sequence of the target gene are not limited. The target gene includes a nucleic acid encoding a protein and a nucleic acid not encoding a protein.

Examples of the target gene include a gene encoding at least one selected from the group consisting of an enzyme, an antibody, an interleukin, a cytokine, a chemokine, a hormone, a growth factor, a transcription factor, a receptor, a transcription-controlling nucleic acid, a non-coding RNA, a viral preparation, a vaccine, a therapeutic protein, a subunit thereof, and a fragment thereof.

The meaning and specific form of the target gene are the same as the meaning and specific form of the target gene described in the description of the cell production method of the present disclosure.

The cell selection method of the present disclosure includes the following (11).

(11) calculating a TAD score indicating a transcriptional activity for each TAD in a genome of a cell, selecting a TAD based on the TAD score, and selecting a cell in which a target gene is present in the selected TAD.

The TAD and the TAD score in (11) have the same meanings as the TAD and the TAD score in (1) of the first search method, and the specific forms and the calculation methods thereof are also the same.

An example of the embodiment of the TAD score is a value obtained by multiplying the density of the gene present in the TAD by the average expression level. The higher the value is, the more preferable the evaluation standard.

Specifically, the confirmation of the presence of the target gene in the TAD is performed, for example, by genome extraction from the cell, production of a sequencing library, library sequencing, mapping of reads onto the genome, mapping of the target gene onto the reads, extraction of reads including the target gene, and measurement of the copy number of the target gene included in the reads.

From the viewpoint of acquiring a long read and the viewpoint of obtaining DNA methylation data, the library sequencing is preferably single-molecule real-time sequencing or nanopore sequencing.

The cell selected by (11) is expected to be a cell in which the target gene is highly expressed because the target gene is present in the TAD having a relatively high transcriptional activity.

An example of the embodiment of (11) includes selecting a cell in which the target gene is present in multiple copies (that is, the number of copies of the coding sequence is 2 or more) in the selected TAD. This cell is highly likely to highly express the target gene. From the viewpoint of long-term subculture stability of the cell, the number of copies of the target gene present in the selected TAD is preferably not too many, and is preferably 2 copies to 6 copies, more preferably 2 copies to 5 copies, and still more preferably 2 copies to 4 copies.

An example of the embodiment of the cell selection method according to the present disclosure further includes the following (12).

(12) specifying a TAD score-SH, which is a TAD score of a TAD to which at least one known safe harbor in a genome belongs,
determining a threshold value based on at least one TAD score-SH,
selecting a TAD based on the threshold value and the TAD score, and selecting a cell in which a target gene is present in the selected TAD.

The known safe harbor in the genome, the TAD score-SH, and the threshold value in (12) have the same meanings as the known safe harbor in the genome, the TAD score-SH, and the threshold value in (2) of the first search method, and specific forms and specific methods thereof are also the same.

The cell selected by (12) is expected to exhibit a gene expression level that is equal to or higher than that of a cell in which the target gene is inserted into a known safe harbor.

Even in a case where (11) and (12) cannot be clearly distinguished from each other, the cell selection method according to the present disclosure includes (11) and (12) as long as the objects of (11) and (12) are achieved.

An example of the embodiment of the cell selection method according to the present disclosure further includes the following (13).

(13) measuring a methylation rate of a target gene present in the selected TAD, and selecting a cell in which the methylation rate is a low value.

DNA methylation generally suppresses gene expression. Therefore, by performing (13), a cell in which the target gene is highly expressed is selected.

The methylation rate of the target gene can be acquired from data obtained by performing single-molecule real-time sequencing or nanopore sequencing as the library sequencing in (11).

The value of the methylation rate is, for example, methylated cytosine at the CpG site/total cytosine at the CpG site × 100.

The value of the methylation rate may be a value of the entire target gene, a value of a part of the target gene (for example, a value of a sequence encoding a protein), or a value for each range in which the target gene is divided according to the function (for example, a value of each of a promoter region and a sequence encoding a protein). At least one of a minimum value, a maximum value, an average value, or a median value of the methylation rates obtained from the target range is used as a representative value for selecting the cell.

Selecting a cell in which the methylation rate of the target gene is a low value is, for example, selecting a cell in which the methylation rate is relatively low; or selecting a cell in which the methylation rate is lower than a predetermined criterion (for example, 30%, 20%, or 10%).

The cell that is a target of the cell selection method according to the present disclosure is any cell as long as the cell has a genome including the structural unit TAD. Examples of the cell include fungi, yeast, insect cells, mammalian cells, and plant cells. Specific examples of the cell are the same as the cells described in the description of the search method according to the present disclosure.

An example of the embodiment of the cell selection method according to the present disclosure targets mammalian cells. Examples of the mammalian cell include Chinese hamster ovary cells (CHO cells), baby hamster kidney cells (BHK cells), a human embryonic kidney cell line (for example, HEK293 cells), a human retinoblast-derived cell line (for example, PER.C6 cells), a mouse myeloma cell line (for example, NS0 cells and SP2/0 cells), and established cell lines derived from these cells.

An example of the embodiment of the cell selection method according to the present disclosure targets CHO cells. Examples of the CHO cell include a CHO-DG44 cell, a CHO-K1 cell, a CHO-DXB11 cell, a CHOpro3⁻ cell, and an established cell derived from these cells.

An example of the embodiment of the cell selection method according to the present disclosure targets cells differentiated from mammalian cells having a differentiation ability. For example, cells obtained by introducing a target gene into pluripotent stem cells (ES cells, iPS cells, and the like) or multipotent stem cells (mesenchymal stem cells, tissue stem cells, somatic stem cells, and the like) and then differentiating the cells are targeted.

### <Cell>

The present disclosure provides a cell in which an exogenous target gene is incorporated into a genome, in which the target gene is highly expressed.

The cell according to the present disclosure is a cell derived from a Chinese hamster. Examples of the cell derived from a Chinese hamster include fibroblasts, adipocytes, adipose-derived stem cells, bone marrow-derived stem cells, ovary cells, and established cell lines derived from these cells.

An example of the embodiment of the cell derived from a Chinese hamster is a Chinese hamster ovary cell (CHO cell). Examples of the CHO cell include a CHO-DG44 cell, a CHO-K1 cell, a CHO-DXB11 cell, a CHOpro3⁻ cell, and an established cell derived from these cells.

The cell according to the present disclosure is a cell in which a target gene is inserted into at least one region selected from 40 regions shown in Table 1 below in a genome of a cell derived from a Chinese hamster.

The 40 regions shown in Table 1 are regions specified by RefSeq accession numbers and base numbers of Chinese hamsters, that is, "RefSeq ID", "start", and "end" in Table 1.

**[Table 1]**

| ID | RefSeq ID | start | end | TAD start | TAD end |
|---|---|---|---|---|---|
| ID001 | NC_048599.1 | 14,718,000 | 14,719,000 | 14,500,000 | 15,500,000 |
| ID002 | NC_048596.1 | 51,129,000 | 51,130,000 | 50,900,000 | 51,400,000 |
| ID003 | NW_023276806.1 | 59,884,000 | 59,885,000 | 59,800,000 | 60,700,000 |
| ID004 | NC_048595.1 | 36,214,000 | 36,215,000 | 35,700,000 | 36,300,000 |
| ID005 | NC_048600.1 | 114,071,000 | 114,072,000 | 113,400,000 | 114,500,000 |
| ID006 | NC_048600.1 | 91,446,000 | 91,447,000 | 90,900,000 | 91,700,000 |
| ID007 | NW_023276806.1 | 130,298,000 | 130,299,000 | 129,000,000 | 130,800,000 |
| ID008 | NC_048595.1 | 399,476,000 | 399,478,000 | 398,500,000 | 399,800,000 |
| ID009 | NC_048600.1 | 44,678,000 | 44,679,000 | 44,300,000 | 45,900,000 |
| ID010 | NC_048597.1 | 133,640,000 | 133,641,000 | 133,300,000 | 134,100,000 |
| ID011 | NC_048596.1 | 49,747,000 | 49,748,000 | 49,400,000 | 49,900,000 |
| ID012 | NW_023276806.1 | 64,039,000 | 64,040,000 | 63,800,000 | 64,400,000 |
| ID013 | NC_048599.1 | 24,283,000 | 24,284,000 | 23,300,000 | 24,700,000 |
| ID014 | NC_048597.1 | 103,921,000 | 103,922,000 | 103,500,000 | 104,300,000 |
| ID015 | NW_023276807.1 | 98,900,000 | 98,901,000 | 98,200,000 | 99,100,000 |
| ID016 | NC_048596.1 | 52,669,000 | 52,670,000 | 52,400,000 | 53,000,000 |
| ID017 | NC_048595.1 | 4,234,000 | 4,235,000 | 3,800,000 | 4,400,000 |
| ID018 | NC_048600.1 | 128,311,000 | 128,312,000 | 128,000,000 | 128,600,000 |
| ID019 | NC_048598.1 | 101,190,000 | 101,191,000 | 100,500,000 | 102,300,000 |
| ID020 | NC_048596.1 | 74,294,000 | 74,296,000 | 72,800,000 | 74,600,000 |
| ID021 | NC_048597.1 | 135,053,000 | 135,054,000 | 134,100,000 | 135,100,000 |
| ID022 | NW_023276806.1 | 231,799,000 | 231,800,000 | 231,200,000 | 232,000,000 |
| ID023 | NC_048598.1 | 124,775,000 | 124,776,000 | 124,000,000 | 126,000,000 |
| ID024 | NW_023276806.1 | 99,361,000 | 99,364,000 | 99,200,000 | 100,000,000 |
| ID025 | NW_023276806.1 | 65,423,000 | 65,424,000 | 65,300,000 | 66,200,000 |
| ID026 | NW_023276806.1 | 65,250,000 | 65,251,000 | 64,400,000 | 65,300,000 |
| ID027 | NC_048600.1 | 128,983,000 | 128,984,000 | 128,600,000 | 129,300,000 |
| ID028 | NC_048595.1 | 376,666,000 | 376,667,000 | 376,000,000 | 376,900,000 |
| ID029 | NC_048595.1 | 17,007,000 | 17,010,000 | 15,000,000 | 18,400,000 |
| ID030 | NC_048596.1 | 53,710,000 | 53,712,000 | 53,000,000 | 54,900,000 |
| ID031 | NC_048597.1 | 102,218,000 | 102,219,000 | 102,100,000 | 102,700,000 |
| ID032 | NC_048595.1 | 402,863,000 | 402,867,000 | 402,000,000 | 404,000,000 |
| ID033 | NC_048596.1 | 160,455,000 | 160,457,000 | 159,300,000 | 160,900,000 |
| ID034 | NW_023276806.1 | 113,498,000 | 113,499,000 | 113,000,000 | 114,000,000 |
| ID035 | NC_048600.1 | 83,874,000 | 83,875,000 | 83,500,000 | 85,200,000 |
| ID036 | NC_048598.1 | 97,009,000 | 97,010,000 | 96,900,000 | 97,600,000 |
| ID037 | NC_048600.1 | 43,400,000 | 43,401,000 | 43,300,000 | 44,300,000 |
| ID038 | NC_048599.1 | 11,603,000 | 11,606,000 | 11,300,000 | 11,900,000 |
| ID039 | NC_048596.1 | 182,191,000 | 182,192,000 | 181,900,000 | 182,500,000 |
| ID040 | NC_048597.1 | 6,894,000 | 6,898,000 | 5,300,000 | 7,500,000 |

Table 1 also shows the TAD to which each region belongs. The TAD here is a TAD detected in Examples described later. "TAD start" and "TAD end" in Table 1 are boundary coordinates of a TAD acquired in Examples described later. The meanings of "TAD start" and "TAD end" in Table 2 and Table 3 are the same.

The 40 regions shown in Table 1 are regions consisting of regions found by the second search method according to the present disclosure and the vicinity thereof, and are regions in which a gene can be highly expressed.

Therefore, the cell having a genome in which a target gene is inserted into at least one region selected from the 40 regions shown in Table 1 is a cell in which the target gene can be highly expressed.

A preferred example of the cell according to the present disclosure is a cell in which a target gene is inserted into at least one region selected from the group consisting of 40 regions shown in Table 2 below of a genome of a cell derived from a Chinese hamster and a region having a sequence identity of 90% or more with any one of the 40 regions.

The 40 regions shown in Table 2 are regions specified by a RefSeq accession number and a base number of a Chinese hamster, that is, "RefSeq ID", "start", and "end" in Table 2.

**[Table 2]**

| ID | RefSeq ID | start | end | TAD start | TAD end |
|---|---|---|---|---|---|
| ID001 | NC_048599.1 | 14,718,500 | 14,719,000 | 14,500,000 | 15,500,000 |
| ID002 | NC_048596.1 | 51,129,100 | 51,129,300 | 50,900,000 | 51,400,000 |
| ID003 | NW_023276806.1 | 59,884,700 | 59,885,000 | 59,800,000 | 60,700,000 |
| ID004 | NC_048595.1 | 36,214,700 | 36,215,000 | 35,700,000 | 36,300,000 |
| ID005 | NC_048600.1 | 114,071,400 | 114,071,600 | 113,400,000 | 114,500,000 |
| ID006 | NC_048600.1 | 91,446,400 | 91,446,600 | 90,900,000 | 91,700,000 |
| ID007 | NW_023276806.1 | 130,298,700 | 130,298,900 | 129,000,000 | 130,800,000 |
| ID008 | NC_048595.1 | 399,476,900 | 399,477,100 | 398,500,000 | 399,800,000 |
| ID009 | NC_048600.1 | 44,678,000 | 44,678,200 | 44,300,000 | 45,900,000 |
| ID010 | NC_048597.1 | 133,640,300 | 133,640,400 | 133,300,000 | 134,100,000 |
| ID011 | NC_048596.1 | 49,747,500 | 49,747,600 | 49,400,000 | 49,900,000 |
| ID012 | NW_023276806.1 | 64,039,600 | 64,039,800 | 63,800,000 | 64,400,000 |
| ID013 | NC_048599.1 | 24,283,600 | 24,283,900 | 23,300,000 | 24,700,000 |
| ID014 | NC_048597.1 | 103,921,700 | 103,922,000 | 103,500,000 | 104,300,000 |
| ID015 | NW_023276807.1 | 98,900,400 | 98,900,800 | 98,200,000 | 99,100,000 |
| ID016 | NC_048596.1 | 52,669,000 | 52,669,400 | 52,400,000 | 53,000,000 |
| ID017 | NC_048595.1 | 4,234,000 | 4,234,300 | 3,800,000 | 4,400,000 |
| ID018 | NC_048600.1 | 128,311,600 | 128,311,800 | 128,000,000 | 128,600,000 |
| ID019 | NC_048598.1 | 101,190,200 | 101,190,300 | 100,500,000 | 102,300,000 |
| ID020 | NC_048596.1 | 74,294,900 | 74,295,200 | 72,800,000 | 74,600,000 |
| ID021 | NC_048597.1 | 135,053,600 | 135,053,800 | 134,100,000 | 135,100,000 |
| ID022 | NW_023276806.1 | 231,799,700 | 231,799,900 | 231,200,000 | 232,000,000 |
| ID023 | NC_048598.1 | 124,775,600 | 124,775,700 | 124,000,000 | 126,000,000 |
| ID024 | NW_023276806.1 | 99,361,200 | 99,363,200 | 99,200,000 | 100,000,000 |
| ID025 | NW_023276806.1 | 65,423,200 | 65,423,400 | 65,300,000 | 66,200,000 |
| ID026 | NW_023276806.1 | 65,250,100 | 65,250,200 | 64,400,000 | 65,300,000 |
| ID027 | NC_048600.1 | 128,983,800 | 128,983,900 | 128,600,000 | 129,300,000 |
| ID028 | NC_048595.1 | 376,666,100 | 376,666,300 | 376,000,000 | 376,900,000 |
| ID029 | NC_048595.1 | 17,007,000 | 17,009,100 | 15,000,000 | 18,400,000 |
| ID030 | NC_048596.1 | 53,710,800 | 53,711,100 | 53,000,000 | 54,900,000 |
| ID031 | NC_048597.1 | 102,218,700 | 102,219,000 | 102,100,000 | 102,700,000 |
| ID032 | NC_048595.1 | 402,863,400 | 402,866,800 | 402,000,000 | 404,000,000 |
| ID033 | NC_048596.1 | 160,455,800 | 160,456,100 | 159,300,000 | 160,900,000 |
| ID034 | NW_023276806.1 | 113,498,200 | 113,498,300 | 113,000,000 | 114,000,000 |
| ID035 | NC_048600.1 | 83,874,600 | 83,874,800 | 83,500,000 | 85,200,000 |
| ID036 | NC_048598.1 | 97,009,800 | 97,010,000 | 96,900,000 | 97,600,000 |
| ID037 | NC_048600.1 | 43,400,600 | 43,400,900 | 43,300,000 | 44,300,000 |
| ID038 | NC_048599.1 | 11,603,900 | 11,606,000 | 11,300,000 | 11,900,000 |
| ID039 | NC_048596.1 | 182,191,200 | 182,191,300 | 181,900,000 | 182,500,000 |
| ID040 | NC_048597.1 | 6,894,900 | 6,898,000 | 5,300,000 | 7,500,000 |

The 40 regions shown in Table 2 are regions inside the 40 regions shown in Table 1.

The 40 regions shown in Table 2 are regions consisting of regions found by the second search method according to the present disclosure and the vicinity thereof, and are regions in which a gene can be highly expressed.

Therefore, the cell having a genome in which a target gene is inserted into at least one region selected from the 40 regions shown in Table 2 is a cell in which the target gene can be highly expressed.

A more preferred example of the cell according to the present disclosure is a cell in which a target gene is inserted into at least one region selected from the group consisting of 40 regions shown in Table 3 below of a genome of a cell derived from a Chinese hamster and a region having a sequence identity of 90% or more with any one of the 40 regions.

The 40 regions shown in Table 3 are regions specified by a RefSeq accession number and a base number of a Chinese hamster, that is, "RefSeq ID", "start", and "end" in Table 3.

**[Table 3]**

| ID | RefSeq ID | start | end | TAD start | TAD end |
|---|---|---|---|---|---|
| ID001 | NC_048599.1 | 14,718,537 | 14,718,914 | 14,500,000 | 15,500,000 |
| ID002 | NC_048596.1 | 51,129,124 | 51,129,247 | 50,900,000 | 51,400,000 |
| ID003 | NW_023276806.1 | 59,884,736 | 59,884,955 | 59,800,000 | 60,700,000 |
| ID004 | NC_048595.1 | 36,214,754 | 36,214,975 | 35,700,000 | 36,300,000 |
| ID005 | NC_048600.1 | 114,071,453 | 114,071,525 | 113,400,000 | 114,500,000 |
| ID006 | NC_048600.1 | 91,446,475 | 91,446,543 | 90,900,000 | 91,700,000 |
| ID007 | NW_023276806.1 | 130,298,782 | 130,298,868 | 129,000,000 | 130,800,000 |
| ID008 | NC_048595.1 | 399,476,949 | 399,477,087 | 398,500,000 | 399,800,000 |
| ID009 | NC_048600.1 | 44,678,001 | 44,678,194 | 44,300,000 | 45,900,000 |
| ID010 | NC_048597.1 | 133,640,371 | 133,640,374 | 133,300,000 | 134,100,000 |
| ID011 | NC_048596.1 | 49,747,511 | 49,747,523 | 49,400,000 | 49,900,000 |
| ID012 | NW_023276806.1 | 64,039,641 | 64,039,762 | 63,800,000 | 64,400,000 |
| ID013 | NC_048599.1 | 24,283,669 | 24,283,893 | 23,300,000 | 24,700,000 |
| ID014 | NC_048597.1 | 103,921,782 | 103,921,984 | 103,500,000 | 104,300,000 |
| ID015 | NW_023276807.1 | 98,900,416 | 98,900,795 | 98,200,000 | 99,100,000 |
| ID016 | NC_048596.1 | 52,669,001 | 52,669,381 | 52,400,000 | 53,000,000 |
| ID017 | NC_048595.1 | 4,234,084 | 4,234,208 | 3,800,000 | 4,400,000 |
| ID018 | NC_048600.1 | 128,311,615 | 128,311,783 | 128,000,000 | 128,600,000 |
| ID019 | NC_048598.1 | 101,190,261 | 101,190,263 | 100,500,000 | 102,300,000 |
| ID020 | NC_048596.1 | 74,294,973 | 74,295,112 | 72,800,000 | 74,600,000 |
| ID021 | NC_048597.1 | 135,053,691 | 135,053,742 | 134,100,000 | 135,100,000 |
| ID022 | NW_023276806.1 | 231,799,769 | 231,799,836 | 231,200,000 | 232,000,000 |
| ID023 | NC_048598.1 | 124,775,601 | 124,775,631 | 124,000,000 | 126,000,000 |
| ID024 | NW_023276806.1 | 99,361,271 | 99,363,118 | 99,200,000 | 100,000,000 |
| ID025 | NW_023276806.1 | 65,423,238 | 65,423,335 | 65,300,000 | 66,200,000 |
| ID026 | NW_023276806.1 | 65,250,143 | 65,250,198 | 64,400,000 | 65,300,000 |
| ID027 | NC_048600.1 | 128,983,816 | 128,983,850 | 128,600,000 | 129,300,000 |
| ID028 | NC_048595.1 | 376,666,157 | 376,666,240 | 376,000,000 | 376,900,000 |
| ID029 | NC_048595.1 | 17,007,040 | 17,009,047 | 15,000,000 | 18,400,000 |
| ID030 | NC_048596.1 | 53,710,835 | 53,711,073 | 53,000,000 | 54,900,000 |
| ID031 | NC_048597.1 | 102,218,787 | 102,218,939 | 102,100,000 | 102,700,000 |
| ID032 | NC_048595.1 | 402,863,440 | 402,866,729 | 402,000,000 | 404,000,000 |
| ID033 | NC_048596.1 | 160,455,825 | 160,456,061 | 159,300,000 | 160,900,000 |
| ID034 | NW_023276806.1 | 113,498,266 | 113,498,270 | 113,000,000 | 114,000,000 |
| ID035 | NC_048600.1 | 83,874,602 | 83,874,708 | 83,500,000 | 85,200,000 |
| ID036 | NC_048598.1 | 97,009,803 | 97,009,945 | 96,900,000 | 97,600,000 |
| ID037 | NC_048600.1 | 43,400,692 | 43,400,878 | 43,300,000 | 44,300,000 |
| ID038 | NC_048599.1 | 11,603,997 | 11,605,946 | 11,300,000 | 11,900,000 |
| ID039 | NC_048596.1 | 182,191,235 | 182,191,245 | 181,900,000 | 182,500,000 |
| ID040 | NC_048597.1 | 6,894,998 | 6,897,936 | 5,300,000 | 7,500,000 |

The 40 regions shown in Table 3 are regions inside the 40 regions shown in Table 2.

The 40 regions shown in Table 3 are regions found by the second search method according to the present disclosure, and are regions in which a gene is safely and highly expressed.

Therefore, the cell having a genome in which a target gene is inserted into at least one region selected from the 40 regions shown in Table 3 is a cell in which the target gene can be stably highly expressed.

The origin, the size, and the base sequence of the target gene are not limited. The target gene includes a nucleic acid encoding a protein and a nucleic acid not encoding a protein.

Examples of the target gene include a gene encoding at least one selected from the group consisting of an enzyme, an antibody, an interleukin, a cytokine, a chemokine, a hormone, a growth factor, a transcription factor, a receptor, a transcription-controlling nucleic acid, a non-coding RNA, a viral preparation, a vaccine, a therapeutic protein, a subunit thereof, and a fragment thereof.

In a case where the target gene is a nucleic acid encoding a protein, examples of the target protein include at least one selected from the group consisting of an enzyme, an antibody, an interleukin, a cytokine, a chemokine, a hormone, a growth factor, a transcription factor, a receptor, a protein constituting a viral preparation, a vaccine, a therapeutic protein, a subunit thereof, and a fragment thereof.

Examples of the nucleic acid not encoding a protein include a transcription-controlling nucleic acid, a non-coding RNA, and a nucleic acid constituting a viral preparation.

Examples of the non-coding RNA (ncRNA) include microRNA (miRNA), short hairpin RNA (shRNA), small interfering RNA (siRNA), small nuclear RNA (snRNA), ribosomal RNA (rRNA), and transfer RNA (tRNA).

In the present disclosure, the target gene means a target gene that can be expressed in a cell. The target gene has all sequences necessary for the expression of the target gene. In a case where the target gene is a structural gene, the target gene has all sequences necessary for the expression of a protein encoded by the target gene, and includes a coding sequence of the protein and all nucleic acids (for example, a promoter, a transcription terminator, and a polyadenylation sequence) necessary for transcription and translation of the coding sequence in a cell. The target gene may include one copy of a coding sequence of a protein, or may include two or more copies thereof. For example, the target gene may include at least one copy of a coding sequence of each subunit in order to express all subunits of a heteromultimeric protein. For example, the target gene may have at least one copy of a sequence encoding an H chain of an antibody and at least one copy of a sequence encoding an L chain of the antibody.

An example of the embodiment of the cell according to the present disclosure has a genome into which a nucleic acid in which two or more copies of a coding sequence are linked is inserted as a target gene. The cell according to the present embodiment can highly produce a substance encoded by the target gene.

From the viewpoint of long-term subculture stability of the cell, the number of copies of the coding sequence included in the target gene is preferably not too large, and is preferably 2 to 6, more preferably 2 to 5, and still more preferably 2 to 4.

An example of the embodiment of the cell according to the present disclosure has a genome into which a nucleic acid in which coding sequences of all subunits of a heteromultimeric protein are linked at least one copy each is inserted as a target gene. The cell according to the present embodiment can stably produce a heteromultimeric protein.

From the viewpoint of increasing the expression level of the heteromultimeric protein, the target gene in the present embodiment is preferably a nucleic acid in which two or more copies of a set of coding sequences of all subunits are linked. From the viewpoint of long-term subculture stability of the cell, the number of copies of the set included in the target gene is preferably not too large, and is preferably 2 to 6, more preferably 2 to 5, and still more preferably 2 to 4.

The present disclosure provides a method for producing a cell, which is derived from a Chinese hamster and highly expresses a target gene.

The method for producing a cell includes inserting a target gene into at least one region selected from 40 regions shown in Table 1 of a genome of a cell derived from a Chinese hamster.

A preferred example of the method for producing a cell includes inserting a target gene into at least one region selected from 40 regions shown in Table 2 of a genome of a cell derived from a Chinese hamster.

A more preferred example of the method for producing a cell includes inserting a target gene into at least one region selected from 40 regions shown in Table 3 of a genome of a cell derived from a Chinese hamster.

Inserting a target gene into a target region of a genome is possible by a known genome editing technology.

### <Method for producing cell product>

The present disclosure provides a method for producing a cell product having excellent productivity. In the method for producing a cell product according to the present disclosure, by using a cell that highly expresses a target gene, the productivity of a substance (referred to as a "target substance" in the present disclosure) encoded by the target gene is excellent. The cell that highly expresses a target gene is at least one selected from the group consisting of a cell produced by the method for producing a cell according to the present disclosure, a cell sorted by the method for selecting a cell according to the present disclosure, and a cell according to the present disclosure.

In the method for producing a cell product according to the present disclosure, a cell is cultured to express a target gene and produce a target substance. By culturing a cell, a target substance is produced in the cell, and the target substance is accumulated in a culture solution and/or the cell.

A method for culturing a cell and a culture medium composition may be selected according to the type of the cell. Culture conditions (for example, culture scale, cell density, temperature, and CO₂ concentration) may also be selected depending on the type of host cell.

An example of an embodiment of the method for producing a cell product according to the present disclosure includes recovering a target substance from a culture solution. Examples of a method for recovering a target substance from a culture solution include centrifugal separation, filtration, diafiltration, ion exchange chromatography, affinity chromatography, hydrophobic interaction chromatography, gel filtration chromatography, and high-performance liquid chromatography (HPLC). The recovered target substance is used, for example, for producing a pharmaceutical composition.

An example of an embodiment of the method for producing a cell product according to the present disclosure includes recovering a cell in which a target substance is accumulated, from a culture medium. Examples of a method of recovering cells from a culture medium include centrifugation and filtration. The target substance is accumulated inside or on the surface of the cell according to the properties thereof. The recovered cells are, for example, administered, infused, or transplanted into a mammal.

### Examples

Hereinafter, the search method and the like according to the present disclosure will be described in more detail with reference to specific examples. The materials, the treatment procedures, and the like shown in the following specific examples can be appropriately changed as long as the changes do not depart from the gist of the present disclosure. The scope of the search method and the like according to the present disclosure should not be construed as being limited by the following specific examples.

### <Production of Antibody-producing Cell Line>

### [Method]

A plasmid equipped with a gene encoding a known IgG (a total of two antibody subunit genes, one heavy chain gene and one light chain gene) was constructed based on an artificial plasmid having a dihydrofolate reductase (DHFR) gene. Each of the heavy chain gene and the light chain gene includes an hEF-1α promoter, a coding sequence of a fibronectin secretion leader, a coding sequence of an antibody subunit, and a polyA sequence. The fibronectin secretion leader is a signal peptide that induces the secretion of a polypeptide to the extracellular space.

The arrangement order of the two antibody subunit genes on the plasmid is L chain-H chain. Hereinafter, this gene group (L chain-H chain) will be referred to as "GoI".

The plasmid was linearized and introduced into CHO-DG44 cells by electroporation. Static culture was carried out for 14 to 21 days in a culture medium containing methotrexate (MTX) to establish a cell pool of MTX-resistant cells.

One cell was seeded per well in a 96-well plate, and static culture was carried out in an atmosphere of a temperature of 37°C and a CO₂ concentration of 10% (v/v).

The culture supernatant was recovered on the 14th day of culture, and the antibody concentration was measured using a molecular interaction analyzer Octet Qke (Sartorius AG). Clones with higher antibody concentrations were selected, cultured in a 24-well plate, and then cultured in a bioreactor tube for scale-up. Sixty strains of cells having a high antibody concentration were selected.

For the purpose of confirming the long-term subculture stability of the cells, the cells were suspended in a subculture medium and subcultured every 3 days. The subculture was terminated when the total number of cell divisions exceeded 60.

### <Fed-batch culture test>

### [Method]

Sixty antibody-producing cell lines before and after the long-term subculture were each suspended in 40 mL of a basal medium, transferred to a 125 mL flask for shake culture, and subjected to shake culture and feeding at a speed of 140 rpm in an atmosphere of a temperature of 37°C and a CO₂ concentration of 5% (v/v).

From the 3rd day to the 13th day after the start of the culture, a certain amount of the feed culture medium was added every day. Sampling was carried out every 1 to 3 days, and the cell density, the culture solution components, and the antibody concentration were measured. For the measurement, a cell counter Vi-CELL XR (Beckman Coulter, Inc.), a cell culture environment analyzer FLEX2 (NOVA Biomedical Corporation), and a product measurement device Cedex Bio (Roche Diagnostics GmbH) were used.

On the 14th day after the start of the culture, the culture solution was collected, the cells and cell debris were removed using a depth filter (pore size of 0.22 µm) to obtain a culture supernatant. The antibody concentration in the culture supernatant was measured by liquid chromatography using a protein A column.

### [Results]

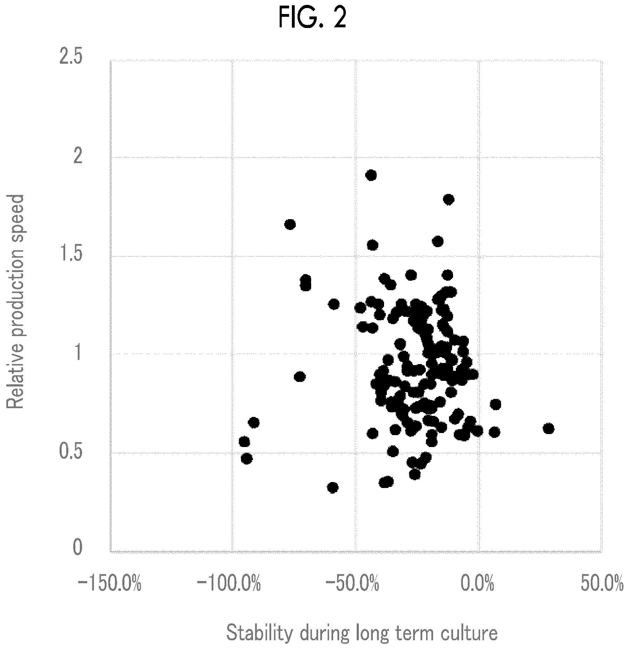

FIG. 2 shows the antibody production performance of the 60 antibody-producing cell lines. In the scatter plot of FIG. 2, the horizontal axis represents the degree of change in the specific production rate before and after the long-term subculture, and the vertical axis represents the relative specific production rate.

From the 60 antibody-producing cell lines, 31 cell lines in which the horizontal axis was -20% or more were selected.

### <Measurement of insertion position of GoI>

### [Method]

The 31 antibody-producing cell lines were shake-cultured for 2 weeks. As the culture medium, a liquid culture medium obtained by adding L-glutamic acid and methotrexate to a serum-free basal culture medium (CD OptiCHO Medium, model number: 12681-011, Thermo Fisher Scientific, Inc.) was used.

4 × 10⁶ cells were collected from the culture medium of each cell, and the genome was extracted using a long-chain genome extraction kit. The chain length of the genome was adjusted and concentrated.

A library was produced from the concentrated genome using a long-read sequencing kit (Ligation Sequencing Kit, model number: SQK-LSK109, Oxford Nanopore Technologies). The sequence of the DNA in the library was read using a nanopore sequencer (model number: M1CCapEx, Oxford Nanopore Technologies).

A fast5 file output from the sequencer was base-called to acquire a fastq file and a corresponding fasta file. From all the reads, reads including the H chain gene and the L chain gene were extracted by a basic local alignment search tool (BLAST), and these were mapped to a CHO genome (GCF_003668045.3) to acquire mapping data (bam file). As the mapping tool, minimap2 was used.

The bam file was visualized using an integrative genomic viewer (IGV) (Broad Institute), and the boundary between the GoI and the genome was comprehensively specified.

Hereinafter, the boundary between the GoI and the genome is referred to as a "junction", and the coordinates thereof are referred to as "junction coordinates".

### [Results]

A total of 226 junctions were found in the 31 antibody-producing cell lines.

### <Measurement of insertion pattern of GoI>

### [Method]

A fast5 file output from the sequencer was base-called to acquire a fastq file. The reads were mapped to a CHO genome (CriGri-PICRH). As the mapping tool, minimap2 was used. Only reads mapped within 6,500 bp from the junction coordinates were extracted.

Using a self-made software "TaulVis", homologous regions with the genome and homologous regions with the GoI in the extracted reads were visualized. Reads in which two junctions and the GoI were read in a connected manner (that is, "junction-GoI-junction") were extracted.

### [Results]

The total number of junctions included in the connected reads was 176.

### <Specification of junctions sandwiching GoI>

### [Method]

The H chain gene and the L chain gene were mapped on the connected reads. As the mapping tool, minimap2 was used. When the complete length of the H chain gene could be mapped, it was counted as the H chain copy number. Similarly, when the complete length of the L chain gene could be mapped, it was counted as the L chain copy number.

Reads in which the H chain gene and the L chain gene were counted as 1 or more copies were extracted, and the junctions included in the extracted reads were specified.

### [Results]

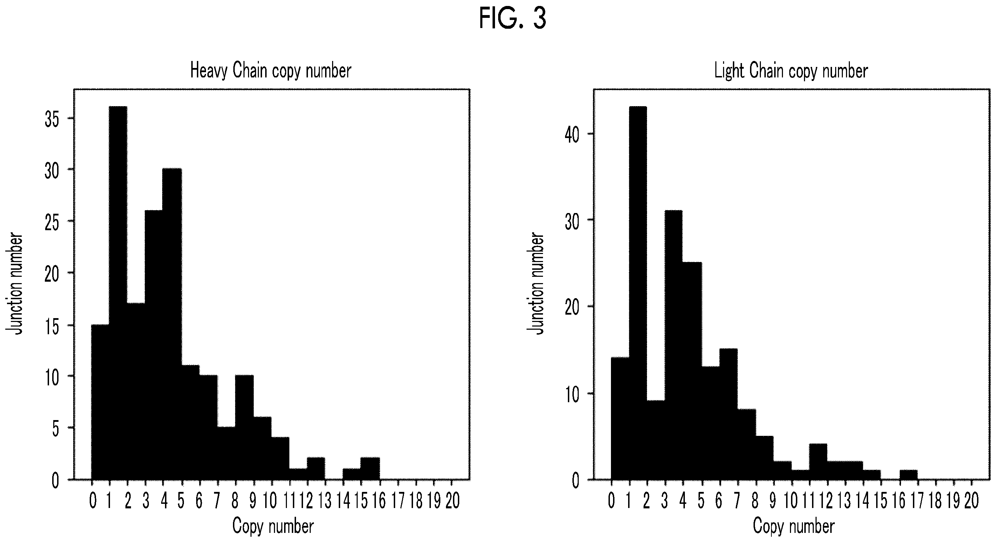

FIG. 3 shows histograms of the copy numbers of the H chain gene and the L chain gene. In the histograms of FIG. 3, the horizontal axis represents the copy number, and the vertical axis represents the number of junctions.

The total number of junctions included in the reads in which the H chain gene and the L chain gene were counted as 1 or more copies was 165.

### <Detection of hypomethylated region>

### [Method]

In the reads in which the H chain gene and the L chain gene were counted as 1 or more copies, methylated cytosine at the CpG site was detected from the above-described fast5 file using a base modification analysis tool (Megalodon, Oxford Nanopore Technologies). The methylation rate (methylated cytosine at CpG site/total cytosine at CpG site × 100) was measured for each of the promoter region, the H chain coding sequence, and the L chain coding sequence, and the arithmetic mean value of the methylation rates was calculated.

### [Results]

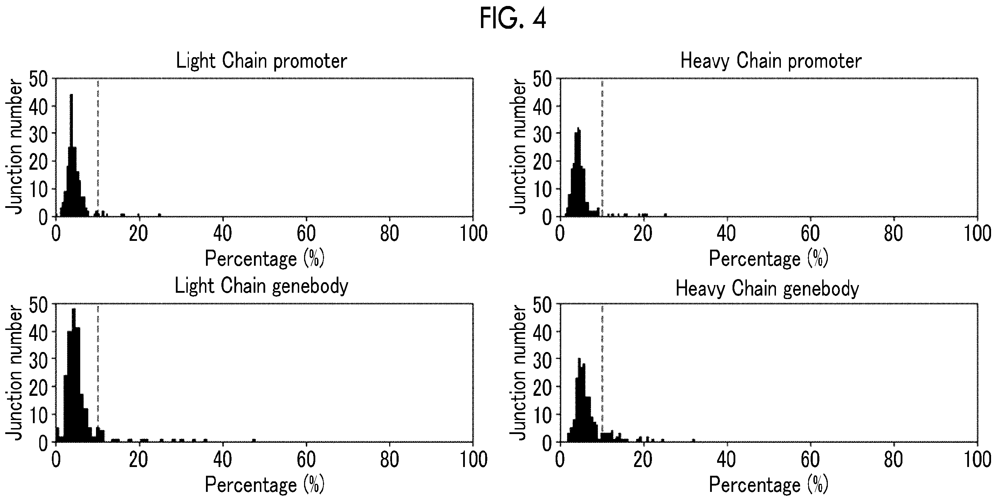

FIG. 4 shows the average methylation rate of each of the promoter region, the H chain coding sequence, and the L chain coding sequence. In the histograms of FIG. 4, the horizontal axis represents the average methylation rate, and the vertical axis represents the number of junctions.

The total number of junctions included in the reads in which all the average methylation rates of the promoter region and the antibody subunit region were 10% or less was 140.

### <Acquisition of boundary coordinates of TAD>

### [Method]

Genome structure data (fastq file, SRRID: SRR12194154) to be subjected to Hi-C analysis was acquired from a paper (William Hilliard, Kelvin H. Lee. Systematic identification of safe harbor regions in the CHO genome through a comprehensive epigenome analysis. Biotechnology and Bioengineering, 2020. https://doi.org/10.1002/bit.27599) in which the genome structure of CHO cells was analyzed.

The fastq file was mapped to the CHO genome (CriGri-PICRH, https://www.ncbi.nlm.nih.gov/datasets/genome/GCF_003668045.3/) using a mapping program bwa (alignment parameters: -A1, -B4, -E50, -L0), and mapping data (bam file) was acquired.

Next, a contact map was acquired from the mapping data (bam file) using the hicBuildMatrix command (binSize: 100000) of HiCExplorer, which is a HiC data analysis and visualization tool.

Next, the boundary coordinates (set of pairs of start point and end point) of the TAD were acquired from the contact map using the hiCFindTADs command (minDepth: 300000, maxDepth: 600000, Step: 100000, minBoundaryDistance: 400000).

### [Results]

The genome structure data acquired from the above paper was data including 281,721,369 reads of paired ends. As a result of mapping all the reads to the CHO genome, there were 150 million reads that were paired, and the contact map for each chromosome was acquired using these reads.

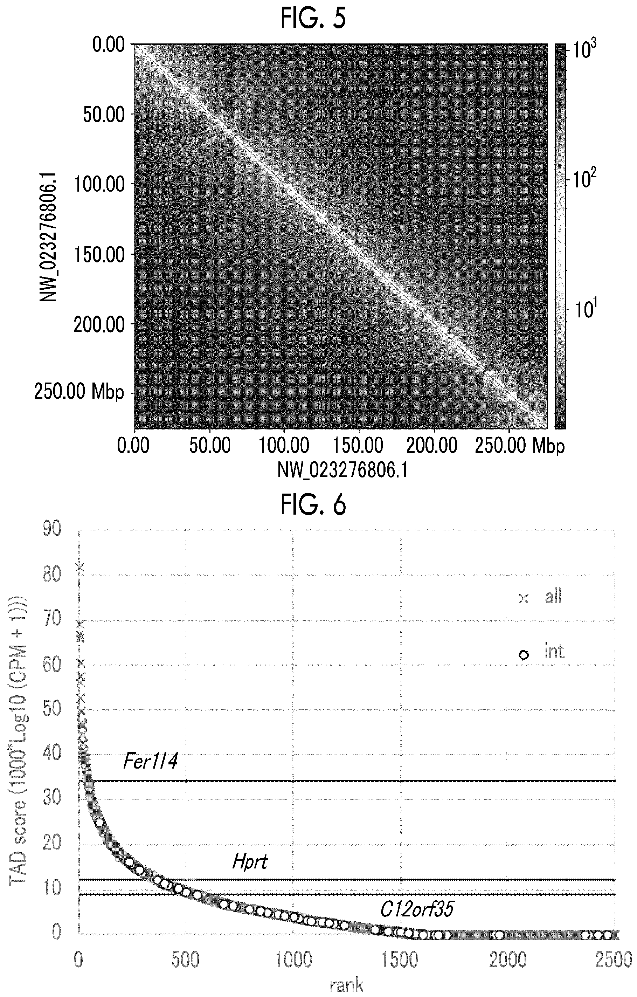

FIG. 5 shows a contact map (expressed as a heat map) of the RefSeq accession number NW_023276806.1.

2502 TADs were detected in the entire CHO genome.

### <Calculation of TAD score>

### [Method]

Based on the coordinate information (GCF_003668045.3_CriGri-PICRH-1.0_genomic.gtf) of the endogenous genes of the CHO cells, the endogenous genes included in each of the 2502 TADs were detected.

RNA-Seq (Takara Bio Inc.) was performed using CHO-DG44 cells as a sample, the mRNA amount of the endogenous genes was quantified, and a value obtained by correcting the mRNA amount to transcripts per million (TPM) was acquired as the expression level of the endogenous genes.

For each of the 2502 TADs, a value obtained by multiplying the density of the endogenous genes by the average expression level of the endogenous genes (average of TPM values after common logarithm transformation) was calculated, and this value was defined as the TAD score.

### [Results]

FIG. 6 shows the distribution of the TAD scores. The "×" in FIG. 6 are the 2502 TAD scores shown in descending order.

### <Specification of TAD score of known safe harbor>

### [Method]

The Fer1l4 locus, the Hprt locus, and the C12orf35 locus are known as safe harbors of CHO cells. The TADs to which these three loci belong were specified, and the TAD scores thereof were specified. The minimum value among the three TAD scores was determined as the threshold value.

### [Results]

The TAD scores of the TADs to which the three loci belong were the following values. The values are shown as three horizontal lines in the distribution diagram of FIG. 6.
Fer1l4 locus = 34.3
Hprt locus = 12.3
C12orf35 locus = 9.0

The threshold value was determined to be 9.0.

### <Selection of junction having high TAD score>

### [Method]

The TAD to which the 140 junctions belong and the TAD score thereof were specified.

Among the 140 junctions, a junction having a TAD score exceeding the threshold value of 9.0 (that is, the TAD score of the C12orf35 locus) was selected.

### [Results]

The "○" in FIG. 6 represents the 140 junctions selected based on the methylation rate.

The TAD scores of 28 junctions among the 140 junctions exceeded 9.0.

### <Detection of junction pair in which chromosomal translocation is not present>

### [Method]

Among the junction pairs flanking the region including one or more copies of the H chain gene and the L chain gene, in a case where it was determined that the junction pair was on the same chromosome based on the junction coordinates and the distance between the junction pairs on the read was 100 kbp or less, it was determined that there was no chromosomal translocation between the junction pairs. Based on this determination criterion, a junction pair in which there was no chromosomal translocation between the junctions was detected.

### [Results]

Among the 28 junctions in which the TAD score exceeded 9.0, 14 junctions were junction pairs in which there was no chromosomal translocation between the junctions.

The seven regions flanked by the seven junction pairs were estimated to be safe harbors of the inserted gene and regions in which the inserted gene was stably highly expressed, by the series of treatments described above.

Table 4 shows the seven regions and the 14 junction coordinates (the "start" and "end" of the seven regions).

**[Table 4]**

| ID | RefSeq ID | start | end | TAD start | TAD end |
|---|---|---|---|---|---|
| ID023 | NC_048598.1 | 124,775,601 | 124,775,631 | 124,000,000 | 126,000,000 |
| ID024 | NW_023276806.1 | 99,361,271 | 99,363,118 | 99,200,000 | 100,000,000 |
| ID029 | NC_048595.1 | 17,007,040 | 17,009,047 | 15,000,000 | 18,400,000 |
| ID032 | NC_048595.1 | 402,863,440 | 402,866,729 | 402,000,000 | 404,000,000 |
| ID034 | NW_023276806.1 | 113,498,266 | 113,498,270 | 113,000,000 | 114,000,000 |
| ID038 | NC_048599.1 | 11,603,997 | 11,605,946 | 11,300,000 | 11,900,000 |
| ID040 | NC_048597.1 | 6,894,998 | 6,897,936 | 5,300,000 | 7,500,000 |

### <Verification of gene expression level of seven regions>

### [Method]

For each of the seven regions shown in Table 4, a plasmid in which homology arms for the seven regions were added to both ends of the inserted gene was produced. The inserted gene was the two antibody subunit genes (L chain-H chain) described above.

The produced plasmid was introduced into CHO cells by electroporation (4D-Nucleofector X unit system, Lonza).

A strain in which the inserted gene was introduced only once into the predetermined region was selected by PCR and a digital PCR system (QX-200, Bio-Rad Laboratories, Inc.) for amplifying the boundary between the genome and the inserted gene.

As a comparative example, a strain in which only one copy of the inserted gene was introduced into the Fer1l4 locus, which is a known safe harbor of CHO cells, was produced in the same manner as described above.

As a comparative example, the linearized inserted gene was introduced into CHO cells by electroporation and randomly integrated into the genome. A strain in which only one copy of the inserted gene was introduced per genome was selected by a digital PCR system.

The cell strain was cultured, and the mRNA amount of each of the H chain and the L chain was quantified by real-time PCR (CFX96, Bio-Rad Laboratories, Inc.).

### [Results]

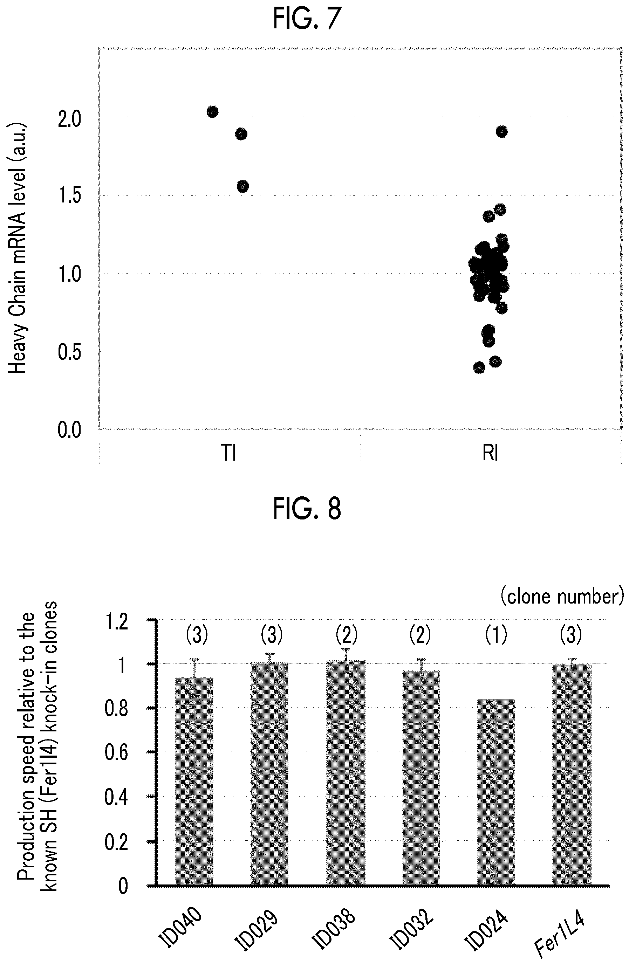

FIG. 7 shows the mRNA amount of the H chain. The vertical axis is the mRNA amount ratio to the average value of the mRNA amounts of 47 strains, which were produced by random integration and in which only one copy of the inserted gene was introduced.

"RI" is 47 strains produced by random integration.

"TI" is a strain in which the gene was inserted into the Fer1l4 locus, a strain in which the gene was inserted into ID029, and a strain in which the gene was inserted into ID040, in this order from the top.

From the results shown in FIG. 7, it can be seen that the gene insertion into the region found by the present embodiment has a higher probability of producing a strain in which the target gene is highly expressed, as compared with the random integration.

FIG. 8 shows the specific production rate of the H chain of the strain in which the gene was inserted into ID040, the strain in which the gene was inserted into ID029, the strain in which the gene was inserted into ID038, the strain in which the gene was inserted into ID032, and the strain in which the gene was inserted into ID024. The specific production rate here is a ratio to the production rate of the strain in which the gene was inserted into the Fer1l4 locus.

The above-described 5 strains had a specific production rate of more than 0.8, and were not inferior to the strain in which the gene was inserted into the Fer1l4 locus.

### <Production of multi-copy insertion strain>

### [Method]

The multi-copy insertion of the coding sequence was attempted in the 7 regions shown in Table 4. For each of the 7 regions, a plasmid in which homology arms for the 7 regions were added to both ends of the inserted gene was produced. The inserted gene is a nucleic acid in which two or four copies of the above-described two antibody subunit genes (L chain-H chain) are linked.

The produced plasmid was introduced into CHO cells by electroporation (4D-Nucleofector X unit system, Lonza).

A strain in which only 2 copies or 4 copies of the inserted gene were introduced into the predetermined region was selected by PCR and a digital PCR system (QX-200, Bio-Rad Laboratories, Inc.) for amplifying the boundary between the genome and the inserted gene.

For the purpose of confirming the long-term subculture stability of the cells, the cells were suspended in a subculture medium and subcultured every 3 days. The subculture was terminated when the total number of cell divisions exceeded 60.

### [Results]

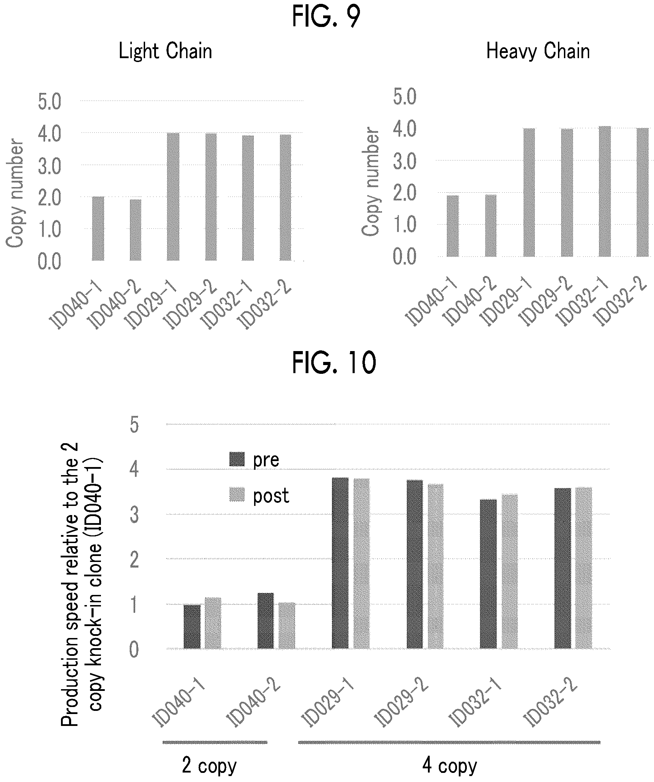

FIG. 9 shows the number of copies of the L chain and the H chain determined by ddPCR. The horizontal axis represents the name of the produced strain, and an identification number of the strain is added after the region ID.

A nucleic acid in which two copies of the L chain-H chain were linked was inserted into the ID040-1 strain and the ID040-2 strain. ID040 was a region in which at least two copies of the L chain-H chain could be inserted.

A nucleic acid in which four copies of the L chain-H chain were linked was inserted into the ID029-1 strain and the ID029-2 strain. ID029 was a region in which at least four copies of the L chain-H chain could be inserted.

A nucleic acid in which four copies of the L chain-H chain were linked was inserted into the ID032-1 strain and the ID032-2 strain. ID032 was a region in which at least four copies of the L chain-H chain could be inserted.

### <Fed-batch culture test>

### [Method]

The ID040-1 strain, the ID040-2 strain, the ID029-1 strain, the ID029-2 strain, the ID032-1 strain, and the ID032-2 strain were subjected to a culture test.

Each of the six antibody-producing strains before and after the long-term subculture was suspended in 40 mL of a basal medium, transferred to a 125 mL flask for shake culture, and subjected to shake culture and feeding at a temperature of 37°C and a CO₂ concentration of 5% (v/v) in an atmosphere at a speed of 140 rpm.

From the 3rd day to the 13th day after the start of the culture, a certain amount of the feed culture medium was added every day. Sampling was carried out every 1 to 3 days, and the cell density, the culture solution components, and the antibody concentration were measured. For the measurement, a cell counter Vi-CELL XR (Beckman Coulter, Inc.), a cell culture environment analyzer FLEX2 (NOVA Biomedical Corporation), and a product measurement device Cedex Bio (Roche Diagnostics GmbH) were used.

On the 14th day after the start of the culture, the culture solution was collected, the cells and cell debris were removed using a depth filter (pore size of 0.22 µm) to obtain a culture supernatant. The antibody concentration in the culture supernatant was measured by liquid chromatography using a protein A column.

### [Results]

FIG. 10 shows the antibody production performance of the six antibody-producing strains before and after the long-term culture. pre indicates the antibody-producing strain before the long-term culture, and post indicates the antibody-producing strain after the long-term culture. The vertical axis represents the relative specific production rate.

From the results shown in FIG. 10, it can be seen that the antibody-producing strains in which two or four copies of the L chain-H chain are inserted into ID040, ID029, or ID032 can stably produce antibodies without a decrease in the antibody production amount even after the long-term culture. It can be seen that the strain in which four copies of the L chain-H chain are inserted into ID029 or ID032 has a higher antibody productivity than the strain in which two copies of the L chain-H chain are inserted into ID040, and the antibody production amount increases with an increase in the number of copies of the coding sequence.

All documents, patent applications, and technical standards described in the present specification are incorporated herein by reference to the same extent as in a case of being specifically and individually noted that individual documents, patent applications, and technical standards are incorporated herein by reference.

The disclosure of JP2023-202228 filed on November 29, 2023 is incorporated herein by reference in its entirety.

## Claims

1. A search method for finding a target region on a genome for inserting a target gene, the search method comprising:
the following (1),
wherein the search method finds a TAD as the target region,
(1) calculating, for each TAD included in the genome, a TAD score indicating transcriptional activity, and selecting a TAD based on the TAD score.

2. The search method according to claim 1, further comprising:
the following (2);
(2) for at least one known safe harbor in the genome, identifying a TAD score-SH that is the TAD score of a TAD to which the safe harbor belongs,
determining a threshold value based on at least one TAD score-SH, and
selecting a TAD based on the threshold value and the TAD score.

3. A search method for finding a target region on a genome for inserting a target gene, the search method comprising:
the following (a) to (d),
wherein the search method finds, as the target region, a region identified by a boundary pair P described below,
(a) providing cells having a genome F in which an exogenous gene is integrated and expressing the exogenous gene,
(b) obtaining the genome F from the cells, analyzing the genome F, and identifying a region F that is a region including the exogenous gene and a boundary F that is a boundary between the region F and the genome,
(c) calculating, for each TAD included in the genome, a TAD score indicating transcriptional activity, identifying, for each boundary F, a TAD score-F that is the TAD score of a TAD to which the boundary F belongs, and selecting the boundary F based on the TAD score-F, and
(d) finding, from among the selected boundaries F, a boundary pair P sandwiching the region F.

4. The search method according to claim 3, further comprising:
the following (p),
wherein the (c) is performed for the boundary F selected in the following (p),
(p) measuring a methylation rate of the exogenous gene present in the region F and selecting a boundary F of the region F for which the methylation rate is low.

5. The search method according to claim 3 or 4, further comprising:
the following (q),
wherein the (d) is performed for the boundary F selected in (c) and the following (q),
(q) for at least one known safe harbor in the genome, identifying a TAD score-SH that is the TAD score of a TAD to which the safe harbor belongs,
determining a threshold value based on at least one TAD score-SH, and
selecting the boundary F based on the threshold value and the TAD score-F.

6. The search method according to claim 3 or 4, further comprising:
the following (r),
(r) selecting, in the genome F, a boundary pair P in which the region F between the boundary pair P is not a region formed by chromosomal translocation.

7. The search method according to any one of claims 1 to 4,
wherein the TAD score is a value obtained by multiplying a density of genes present in the TAD by an average expression level.

8. The search method according to claim 1 or 3,
wherein the genome is a genome of a mammalian cell.

9. The search method according to claim 1 or 3,
wherein the genome is a genome of a CHO cell.

10. A method for producing a cell, the method comprising:
finding the TAD by the search method according to claim 1; and
inserting a target gene into an inside of the TAD in a genome having the TAD.

11. A method for producing a cell, the method comprising:
finding a region identified by the boundary pair P by the search method according to claim 3; and
inserting a target gene into a region within ±10 kbp before and after the region in a genome having the region.

12. The method for producing a cell according to claim 10 or 11,
wherein the target gene is a gene encoding at least one selected from the group consisting of an enzyme, an antibody, an interleukin, a cytokine, a chemokine, a hormone, a growth factor, a transcription factor, a receptor, a transcription-regulatory nucleic acid, a non-coding RNA, a viral preparation, a vaccine, a therapeutic protein, subunits thereof, and fragments thereof.

13. A method for selecting a cell, which is a method for selecting a cell expressing a target gene, the method comprising:
the following (11),
(11) calculating, for each TAD included in a genome of a cell, a TAD score indicating transcriptional activity; selecting a TAD based on the TAD score; and selecting a cell in which the target gene is present in the selected TAD.

14. The method for selecting a cell according to claim 13, the method further comprising:
the following (12),
(12) for at least one known safe harbor in the genome, identifying a TAD score-SH that is the TAD score of a TAD to which the safe harbor belongs,
determining a threshold based on at least one TAD score-SH; selecting a TAD based on the threshold and the TAD score, and
selecting a cell in which the target gene is present in the selected TAD.

15. The method for selecting a cell according to claim 13 or 14, the method further comprising:
the following (13),
(13) measuring a methylation rate of the target gene present in the selected TAD, and selecting a cell having a low methylation rate.

16. The method for selecting a cell according to claim 13 or 14,
wherein the TAD score is a value obtained by multiplying a density of genes present in the TAD by an average expression level.

17. The method for selecting a cell according to claim 13,
wherein the cell is a mammalian cell.

18. The method for selecting a cell according to claim 13,
wherein the cell is a CHO cell.

19. The method for selecting a cell according to claim 13,
wherein the target gene is a gene encoding at least one selected from the group consisting of an enzyme, an antibody, an interleukin, a cytokine, a chemokine, a hormone, a growth factor, a transcription factor, a receptor, a transcription-regulatory nucleic acid, a non-coding RNA, a viral preparation, a vaccine, a therapeutic protein, subunits thereof, and fragments thereof.

20. A cell derived from a Chinese hamster,
wherein a target gene is inserted into at least one region selected from 40 regions shown in Table 1 below
**[Table 1]**
| ID | RefSeq ID | start | end |
|---|---|---|---|
| ID001 | NC_048599.1 | 14,718,000 | 14,719,000 |
| ID002 | NC_048596.1 | 51,129,000 | 51,130,000 |
| ID003 | NW_023276806.1 | 59,884,000 | 59,885,000 |
| ID004 | NC_048595.1 | 36,214,000 | 36,215,000 |
| ID005 | NC_048600.1 | 114,071,000 | 114,072,000 |
| ID006 | NC_048600.1 | 91,446,000 | 91,447,000 |
| ID007 | NW_023276806.1 | 130,298,000 | 130,299,000 |
| ID008 | NC_048595.1 | 399,476,000 | 399,478,000 |
| ID009 | NC_048600.1 | 44,678,000 | 44,679,000 |
| ID010 | NC_048597.1 | 133,640,000 | 133,641,000 |
| ID011 | NC_048596.1 | 49,747,000 | 49,748,000 |
| ID012 | NW_023276806.1 | 64,039,000 | 64,040,000 |
| ID013 | NC_048599.1 | 24,283,000 | 24,284,000 |
| ID014 | NC_048597.1 | 103,921,000 | 103,922,000 |
| ID015 | NW_023276807.1 | 98,900,000 | 98,901,000 |
| ID016 | NC_048596.1 | 52,669,000 | 52,670,000 |
| ID017 | NC_048595.1 | 4,234,000 | 4,235,000 |
| ID018 | NC_048600.1 | 128,311,000 | 128,312,000 |
| ID019 | NC_048598.1 | 101,190,000 | 101,191,000 |
| ID020 | NC_048596.1 | 74,294,000 | 74,296,000 |
| ID021 | NC_048597.1 | 135,053,000 | 135,054,000 |
| ID022 | NW_023276806.1 | 231,799,000 | 231,800,000 |
| ID023 | NC_048598.1 | 124,775,000 | 124,776,000 |
| ID024 | NW_023276806.1 | 99,361,000 | 99,364,000 |
| ID025 | NW_023276806.1 | 65,423,000 | 65,424,000 |
| ID026 | NW_023276806.1 | 65,250,000 | 65,251,000 |
| ID027 | NC_048600.1 | 128,983,000 | 128,984,000 |
| ID028 | NC_048595.1 | 376,666,000 | 376,667,000 |
| ID029 | NC_048595.1 | 17,007,000 | 17,010,000 |
| ID030 | NC_048596.1 | 53,710,000 | 53,712,000 |
| ID031 | NC_048597.1 | 102,218,000 | 102,219,000 |
| ID032 | NC_048595.1 | 402,863,000 | 402,867,000 |
| ID033 | NC_048596.1 | 160,455,000 | 160,457,000 |
| ID034 | NW_023276806.1 | 113,498,000 | 113,499,000 |
| ID035 | NC_048600.1 | 83,874,000 | 83,875,000 |
| ID036 | NC_048598.1 | 97,009,000 | 97,010,000 |
| ID037 | NC_048600.1 | 43,400,000 | 43,401,000 |
| ID038 | NC_048599.1 | 11,603,000 | 11,606,000 |
| ID039 | NC_048596.1 | 182,191,000 | 182,192,000 |
| ID040 . | NC_048597.1 | 6,894,000 | 6,898,000 |

21. The cell according to claim 20,
wherein the cell derived from a Chinese hamster is a CHO cell.

22. The cell according to claim 20 or 21,
wherein the target gene is a gene encoding at least one selected from the group consisting of an enzyme, an antibody, an interleukin, a cytokine, a chemokine, a hormone, a growth factor, a transcription factor, a receptor, a transcription-regulatory nucleic acid, a non-coding RNA, a viral preparation, a vaccine, a therapeutic protein, subunits thereof, and fragments thereof.

23. A method for producing a cell product, comprising:
culturing a cell produced by the method for producing a cell according to claim 10 or 11 to express the target gene.

24. A method for producing a cell product, comprising:
culturing a cell selected by the cell selection method according to claim 13 or 14 to express the target gene.

25. A method for producing a cell product, comprising:
culturing the cell according to claim 20 or 21 to express the target gene.
